# EUROPEAN PATENT APPLICATION

(11) **EP 4 356 932 A1**
(43) Date of publication of application: **24.04.2024**
(21) Application number: 22824303.6
(22) Date of filing: 17.06.2022
(51) Int. Cl.: A61K 48/00, A61K 31/57, G01N 33/50, A61P 25/28, A61P 3/00

(54) **METHOD AND DRUG FOR TREATING NEURONAL CEROID LIPOFUSCINOSIS**

(30) Priority: 17.06.2021 CN 202110674245
(71) Applicant: Tsinghua University, Beijing 100084 (CN)
(72) Inventor: ZHI, Yun, Beijing 100084 (CN); DING, Sheng, Beijing 100084 (CN); MA, Tianhua, Beijing 100084 (CN)
(74) Representative: Cabinet Becker et Associés
(86) International application number: PCT/CN2022/099308
(87) International publication number: WO 2022/262830

(57) **Abstract**

A method for treating a disease associated with cell autophagy functional deficiency and/or lysosomal functional deficiency, such as neurodegenerative diseases, by inhibiting KEAP1 activity and/or enhancing the expression of nuclear erythroid 2-related factor 2 (NRF2) and/or the downstream genes thereof. The expression of NRF2 and/or the downstream genes thereof is enhanced with a KEAP1 inhibitor, a KEAP1 gene knockdown, and/or an NRF2 activator. The method and product can increase the acidic environment in the lysosomes of neurons, increase the enzymatic activity of cathepsins, decrease the abnormal storage of proteins, protect mitochondrial homeostasis in neurons, and increase the capability of mitochondria to generate ATP.

## Description

### Technical Field

The present disclosure relates to a method and medicament for the treatment of neurodegenerative diseases such as neuronal ceroid lipofuscinosis, in particular juvenile neuronal ceroid lipofuscinosis (JNCL).

### Background

Neuronal ceroid lipofuscinosis (NCL) is a rare inherited neurological disease, which belongs to the family of lysosomal storage diseases (LSD), most of which are autosomal recessive. Symptoms of NCLs occur mostly in infancy and childhood, and most patients present with a progressive neurodegenerative clinical course. As a typical NCL disease, juvenile neuronal ceroid lipofuscinosis (JNCL), also known as Batten disease, is one of the most common inherited juvenile neurological diseases, and is caused by *CLN3* gene mutation. The cells of JNCL patients show obvious lysosomal defect features, and many substances such as subunit c of mitochondrial ATP synthase (SCMAS), lipoprotein and glycoprotein cannot be degraded normally and thus gradually accumulate in lysosomes. Neurons are one of the most affected cell types by CLN3 mutations because they are highly lysosomal dependent for substance metabolism. Patients exhibit severe visual impairment around the age of 2-10 years, followed by epilepsy, progressive motor deficits and cognitive decline, and usually die of the disease between the ages of 20-30 years. Currently, there is no radical cure for JNCL, and all clinical treatments are symptomatic and palliative.

Lysosome is an organelle that plays a key role in the degradation and recycling of intracellular and intracellular substances (Luzio et al., 2009; Mizushima and Komatsu, 2011). In order to maintain cellular homeostasis, a network consisting of lysosomes, endosomes, autophagosomes, and other cellular components performs the function of degrading materials inside and outside the cell (Saftig and Klumperman, 2009). Changes in lysosomal acidity affect the activity of a variety of enzymes in the lumen, thereby disrupting their substrate clearance processes, leading to the development of a variety of neurodegenerative diseases, including NCL (Song et al., 2020).

Previous studies have shown that CLN3 protein is mainly located on the lysosomal membrane of cells, but its function in cells remains unclear. Studies on JNCL disease models *in vitro* and *in vivo* have shown that it has lysosomal damage phenotypes such as dysregulation of lysosomal acidity, reduced protease activity in lysosome, and autophagy defects. Abnormal autophagy and lysosome function is one of the important causes of aging and neurodegenerative diseases, however, the role of autophagy and lysosomes in the mechanisms of aging and the pathogenesis of neurodegenerative diseases remains to be studied and explored.

Studies have shown that activation of autophagy by promoting autophagy initiation does not produce a therapeutic effect on CLN3 mutated cells. Currently, the most classical autophagy-inducing compounds are small molecules that target the mTORCl signaling pathway, such as Rapamycin. Treatment of *Cln3*^{*Δex7*/*8*} cells with mTORC1 inhibitors increases the formation of autophagosomes while the formed autophagosomes cannot be effectively degraded by lysosomes and accumulate within cells (Centa et al., 2020), and the disease phenotype cannot be effectively alleviated.

Thus, there remains a need for effective treatments for neurodegenerative diseases such as JNCL.

### SUMMARY OF THE INVENTION

One aspect of the present disclosure relates to a method for treating a disease associated with autophagy functional deficiency and/or lysosomal functional deficiency, the method comprising a process of inhibiting the activity of Kelch-like ECH-associated protein 1 (KEAP1), and/or enhancing the activity of nuclear erythroid 2-related factor 2 (NRF2) and/or the downstream proteins thereof.

The method according to the present disclosure, the disease associated with autophagy functional deficiency and/or lysosomal functional deficiency is a neurodegenerative disease.

The method according to the present disclosure, the neurodegenerative disease is selected from the group consisting of amyotrophic lateral sclerosis (ALS), Alzheimer's dementia, Alexander disease, Alper's disease, ataxia-telangiectasia, bovine spongiform encephalopathy (BSE), Canavan disease, Cockayne syndrome, corticobasal degeneration, Creutzfeldt-Jakob disease, Huntington's disease, HIV-related dementia, Kennedy's disease, Krabbedisease, dementia with Lewy bodies, Machado-Joseph disease (spinocerebellar ataxias 3), multiple sclerosis, multiple system atrophy, neurospirillosis, Parkinson's disease, Péme's disease, Pick's disease, primary lateral sclerosis, Prion's disease, Refsum's disease, Sandhoff disease, Hield's disease, schizophrenia, Spielmeyer-Vogt-Sjogren-Batten disease, spinocerebellar ataxias, spinal muscular atrophy, or neuronal ceroid lipofuscinosis (NCL).

In some specific embodiments, the neurodegenerative disease is selected from Parkinson's disease, Alzheimer's disease, Huntington's disease, Kennedy's disease, amyotrophic lateral sclerosis, primary lateral sclerosis, multiple sclerosis, frontotemporal dementia, or neuronal ceroid lipofuscinosis (NCL).

In some preferred embodiments, the neurodegenerative disease is preferably neuronal ceroid lipofuscinosis, more preferably juvenile neuronal ceroid lipofuscinosis (JNCL).

The therapeutic method according to the present disclosure, the disease is caused by a mutation in CLN3 gene, such as caused by *CLN3*^{*Δex7*/*8*}, a deletion mutation in exons 7 and 8 of CLN3 gene, wherein the deletion mutation is about 1.02 kb.

The therapeutic method according to the present disclosure, wherein the expression of KEAP1 gene is silenced or knocked down with siRNA, sgRNA or vectors constructed with shRNA, thereby enhancing the activity of NRF2 and/or the downstream proteins thereof.

The therapeutic method according to the present disclosure, wherein the expression of NRF2 and/or the downstream genes thereof is enhanced with a KEAP1 inhibitor and/or NRF2 activator, thereby enhancing the activity of NRF2 and/or the downstream proteins thereof.

The therapeutic method according to the present disclosure, wherein the KEAP1 inhibitor and/or NRF2 activator is selected from reagents covalently reacting with cysteine residues on KEAP1, including cysteine at the positon on 151, 272 and/or 288 on KEAP1.The therapeutic method according to the present disclosure, wherein the KEAP1 inhibitor and/or NRF2 activator is selected from the group consisting of Carvedilol (CAS: 72956-09-3), ketoconazole (CAS: 65277-42-1), GANT61, CAS (500579-04-4), protriptyline hydrochloride (CAS: 1225-55-4), LP 44 (CAS: 824958-12-5), Doxepin HCI (CAS: 1229-29-4), dimethyl fumarate (DMF), acetyl-11-keto-β-boswellic acid (AKBA), isothiocyanates, bardoxolone (CDDO), bardoxolonemethyl (CDDO-Me) and the derivatives or analogues thereof, or selected from one or more of α, β-unsaturated carbonyl compounds, phenols and polyphenolic compounds.

The therapeutic method according to the present disclosure, wherein the KEAP1 inhibitor is Compound G, namely (Z)-guggulsterone, having the formula:

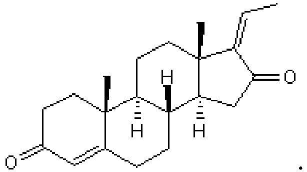

Another aspect of the present disclosure relates to the use of a KEAP1 inhibitor and/or NRF2 activator in the manufacture of a medicament for inhibiting the activity of KEAP1, and/or enhancing the activity of nuclear erythroid 2-related factor 2 (NRF2) and/or the downstream proteins thereof to treat diseases associated with autophagy functional deficiency and/or lysosomal functional deficiency.

The use according to the present disclosure, wherein the disease associated with autophagy functional deficiency and/or lysosomal functional deficiency is a neurodegenerative disease.

The use according to the present disclosure, wherein the neurodegenerative disease is selected from the group consisting of amyotrophic lateral sclerosis (ALS), Alzheimer's dementia, Alexander disease, Alper's disease, ataxia-telangiectasia, bovine spongiform encephalopathy (BSE), Canavan disease, Cockayne syndrome, neurospirillosis, Parkinson's disease, Péme's disease, Pick's disease, primary lateral sclerosis, Prion's disease, Refsum's disease, Sandhoff disease, Hield's disease, schizophrenia, Spielmeyer-Vogt-Sjogren-Batten disease, spinocerebellar ataxias, spinal muscular atrophy, or neuronal ceroid lipofuscinosis (NCL).

In some preferred embodiments, wherein the neurodegenerative disease is preferably neuronal ceroid lipofuscinosis, more preferably juvenile neuronal ceroid lipofuscinosis (JNCL).

The use according to the present disclosure, wherein the disease is caused by a mutation in CLN3 gene, such as caused by *CLN3*^{*Δex7*/*8*}, a deletion mutation in exons 7 and 8 of CLN3 gene, wherein the deletion mutation is about 1.02 kb.

The use according to the present disclosure, wherein the expression of KEAP1 gene is silenced or knocked down with siRNA, sgRNA or vectors constructed with shRNA, thereby enhancing the activity of NRF2 and/or the downstream proteins thereof.

The use according to the present disclosure, wherein the expression of NRF2 and/or the downstream genes thereof is enhanced with a KEAP1 inhibitor and/or NRF2 activator, thereby enhancing the activity of NRF2 and/or the downstream proteins thereof.

The use according to the present disclosure, wherein the KEAP1 inhibitor and/or NRF2 activator is selected from reagents covalently reacting with cysteine residues on KEAP1, including cysteine at the positon 151, 272 and/or 288 on KEAP1.

The use according to the present disclosure, wherein the KEAP1 inhibitor and/or NRF2 activator is selected from the group consisting of Carvedilol (CAS: 72956-09-3), ketoconazole (CAS: 65277-42-1), GANT61, CAS (500579-04-4), protriptyline hydrochloride (CAS: 1225-55-4), LP 44 (CAS: 824958-12-5), Doxepin HCI (CAS: 1229-29-4), dimethyl fumarate (DMF), acetyl-11-keto-β-boswellic acid (AKBA), isothiocyanates, bardoxolone (CDDO), bardoxolonemethyl (CDDO-Me) and the derivatives or analogues thereof, or selected from one or more of α, β-unsaturated carbonyl compounds, phenols and polyphenolic compounds.

The use according to the present disclosure, wherein the KEAP1 inhibitor is Compound G, namely (Z)-guggulsterone, having the formula:

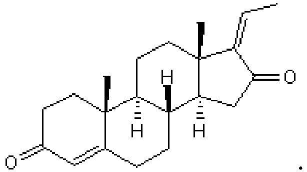

Yet another aspect of the present disclosure relates to a pharmaceutical composition for treating a disease associated with autophagy functional deficiency and/or lysosomal functional deficiency, comprising a therapeutically effective amount of one or more of KEAP1 inhibitor and/or NRF2 activator, and a pharmaceutically acceptable carrier and/or excipient.

The pharmaceutical composition according to the present disclosure, wherein the KEAP 1 inhibitor and/or NRF2 activator is selected from reagents covalently reacting with cysteine residues on KEAP1, including cysteine at the positon 151, 272 and/or 288 on KEAP1.

The pharmaceutical composition according to the present disclosure, wherein the KEAP1 inhibitor and/or NRF2 activator is selected from the group consisting of Carvedilol (CAS: 72956-09-3), ketoconazole (CAS: 65277-42-1), GANT61, CAS (500579-04-4), protriptyline hydrochloride (CAS: 1225-55-4), LP 44 (CAS: 824958-12-5), Doxepin HCI (CAS: 1229-29-4), dimethyl fumarate (DMF), acetyl-11-keto-β-boswellic acid (AKBA), isothiocyanates, bardoxolone (CDDO), bardoxolonemethyl (CDDO-Me) and the derivatives or analogues thereof, or selected from one or more of α, β-unsaturated carbonyl compounds, phenols and polyphenolic compounds.

The pharmaceutical composition according to the present disclosure, wherein the KEAP1 inhibitor is Compound G, namely (Z)-guggulsterone, having the formula:

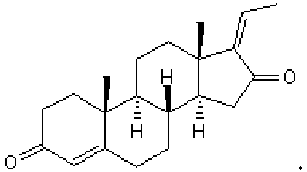

Still another aspect of the present disclosure relates to a kit for treating a disease associated with autophagy functional deficiency and/or lysosomal functional deficiency, comprising a reagent for inhibiting KEAP1 gene expression.

The kit according to the present disclosure, it comprises siRNA, sgRNA or a vector constructed with shRNA for silencing or knocking down KEAP1 gene to inhibit the expression of KEAP1 gene.

Another aspect of the present disclosure relates to a method for alleviating or eliminating the differentiation defect of neural stem cells into neurons in a subject, comprising administering a medicament inhibiting KEAP1 activity and/or enhancing the activity of NRF2 and/or the downstream proteins thereof, wherein the subject is suffering from a disease associated with autophagy functional deficiency and/or lysosomal functional deficiency.

The method according to the present disclosure, wherein the disease is caused by a mutation in CLN3 gene, such as caused by a deletion mutation in exons 7 and 8 of CLN3 gene, the disease is preferably JNCL.

The method according to the present disclosure, wherein the medicament is selected from one or more of the following medicaments: Compound G, namely (Z)-guggulsterone, Carvedilol (CAS: 72956-09-3), Ketoconazole (CAS: 65277-42-1), GANT61, CAS (500579-04-4), Protriptyline hydrochloride (CAS: 1225-55-4), LP 44 (CAS: 824958-12-5), Doxepin HCl (CAS: 1229-29-4), dimethyl fumarate (DMF), acetyl-11-keto-β-boswellic acid (AKBA), isothiocyanates, bardoxolone (CDDO), bardoxolonemethyl (CDDO-Me) and the derivatives or analogues thereof, or selected from other α, β-unsaturated carbonyl compounds, phenols and polyphenolic compounds.

The method according to the present disclosure, wherein the medicament is preferably Compound G, namely (Z)-guggulsterone, having the formula:

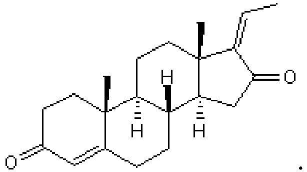

Another aspect of the present disclosure relates to a system for screening substances activating autophagic flux and/or enhancing lysosomal function, wherein the system comprises a Tandem LC3 reporter system.

The screening system according to the present disclosure, wherein when autophagosomes with the dual fluorescently labeled LC3 is located in the cytoplasm, the autophagosomes present, for example red and green, dual fluorescence while when autophagosomes fuse with lysosomes to form an autolysosome, the autophagosomes only present, for example red, single fluorescence.

The screening system according to the present disclosure comprises an NRK cell line stably expressing tandem LC3.

Yet another aspect of the present disclosure relates to a method for screening substances activating autophagic flux and/or enhancing lysosomal function, wherein the aforementioned screening system is used, if substances to be screened increases both the number of dual fluorescence and single fluorescence labeled autophagosomes and the number of autolysosomes in the reporter system, then the substance is a target substance activating autophagic flux and/or enhancing lysosomal function.

Still another aspect of the present disclosure relates to an *in vitro* model based on neural stem cells or neurons, wherein the model comprises a deletion mutation of exons 7 and 8 of the CLN3 gene, namely *CLN3*^{*Δex7*/}*⁸.*

Another aspect of the present disclosure relates to use of the *in vitro* model for screening compounds inducing autophagy and/or verifying the effect of said compounds.

The present disclosure, by inhibiting the expression of KEAP1 gene or activating the expression of NRF2, directly or indirectly activates or enhances the expression of NRF2 and/or the downstream genes thereof, increases the lysosomal acidic environment of neurons and the enzymatic activity of cathepsins in neurons, decreasse the abnormal storage of proteins, protects mitochondrial homeostasis in neurons, and increases the ability of mitochondria to produce ATP, thereby achieving the effect of preventing or treating neurodegenerative diseases associated with autophagy functional deficiency and/or lysosomal functional deficiency.

### DETAILED DESCRIPTION

### DEFINITIONS

To facilitate an understanding herein, certain terms are first defined. Additional definitions are set forth throughout the detailed description.

As used herein, the terms "subject" and "patient" are used interchangeably and refer to a mammal in need of treatment, such as pets (e.g., dogs, cats, and the like), livestock (e.g., cows, pigs, horses, sheep, goats, and the like), and laboratory animals (e.g., rats, mice, guinea pigs, and the like). Typically, the subject is a human in need of treatment.

The term "treatment/treating" refers to obtaining a desired pharmacological and/or physiological effect. The effect may be therapeutic, which includes achieving, partially or substantially, one or more of the following: partially or completely reducing the severity of a disease, disorder, or syndrome; alleviating or improving a clinical symptom or indicator associated with the disorder; and delaying, inhibiting, or reducing the likelihood of progression of the disease, disorder, or syndrome.

The compounds of the present disclosure may be used to treat neurodegenerative disorders. Examples of neurodegenerative disorders include, but are not limited to: amyotrophic lateral sclerosis (ALS), Alzheimer's dementia, Alexander disease, Alper's disease, ataxia-telangiectasia, bovine spongiform encephalopathy (BSE), Canavan disease, Cockayne syndrome, corticobasal degeneration, Creutzfeldt-Jakob disease, Huntington's disease, HIV-related dementia, Kennedy's disease, Krabbedisease, dementia with Lewy bodies, Machado-Joseph disease (spinocerebellar ataxias 3), multiple sclerosis, multiple system atrophy, neurospirillosis, Parkinson's disease, Péme's disease, Pick's disease, primary lateral sclerosis, Prion's disease, Refsum's disease, Sandhoff disease, Hield's disease, schizophrenia, Spielmeyer-Vogt-Sjogren-Batten disease, spinocerebellar ataxias, spinal muscular atrophy, neuronal ceroid lipofuscinosis (NCL), or Batten disease.

The small molecule substances of the present disclosure can be administered to a subject as such or as part of a pharmaceutical composition. As used herein, a "pharmaceutical composition" refers to a product of one or more of the active ingredients described herein with other chemical components, such as physiologically suitable carriers and excipients. The purpose of a pharmaceutical composition is to facilitate administration of a compound to an organism.

Administration of the compound described herein, or a pharmaceutically acceptable salt thereof, or other forms, to a subject may be performed using any suitable delivery method, including topical, enteral, parenteral, transdermal, transmucosal, via inhalation, intracisternal, epidural, intravaginal, intravenous, intramuscular, subcutaneous, intradermal, and intravitreal administration of a compound described herein, or a pharmaceutically acceptable salt thereof, to a subject. Administration of a compound described herein, or a pharmaceutically acceptable salt thereof, or other forms, to a subject further includes topical, enteral, parenteral, transdermal, transmucosal, via inhalation, intracisternal, epidural, intravaginal, intravenous, intramuscular, subcutaneous, intradermal, and intravitreal administration of a compound that can be metabolized to the compound described herein, or a pharmaceutically acceptable salt thereof, or other forms, to a subject in or on its body surface.

The pharmaceutical compositions of the present disclosure may be manufactured by processes well known in the art, e.g. by conventional mixing, dissolving, granulating, dragee-making, levigating, emulsifying, encapsulating, entrapping or lyophilizing.

The compound described herein, or a pharmaceutically acceptable salt thereof, can be administered systemically (e.g. orally) in combination with a pharmaceutically acceptable carrier, such as an inert diluent or an assimilable edible carrier. They may be enclosed in hard or soft shell gelatin capsules, may be compressed into tablets, or may be incorporated directly with the patient's diet. For oral therapeutic administration, the compounds, substances, or pharmaceutically acceptable salts thereof, or other forms described herein, may be combined with one or more excipients and administered in the form of ingestible tablets, buccal tablets, lozenges, capsules, elixirs, suspensions, syrups, or oblaat, and the like.

The tablets, lozenges, pills, capsules and the like may include the following: binders such as gum tragacanth, acacia, corn starch and gelatin; excipients such as dicalcium phosphate; disintegrating agents such as corn starch, potato starch, alginic acid and the like; lubricants, such as magnesium stearate; sweetening agents such as sucrose, fructose, lactose and aspartame; and flavoring agents.

Exemplary pharmaceutical dosage forms for intravenous, intramuscular, subcutaneous, intradermal, etc., injection or infusion include sterile aqueous solutions or dispersions and sterile powders comprising active ingredients, and the sterile powders is suitable for the extemporaneous preparation of sterile injectable or infusible solutions or dispersions. The final dosage form should be sterile, fluid and stable under the conditions of manufacture and storage.

Sterile injectable solutions can be prepared by incorporating the active ingredient in the required amount in the appropriate solvent together with various of the other required ingredients enumerated herein, followed by sterilization by filtration. In the case of the preparation of sterile powders for sterile injectable solutions, the preferred methods of preparation may be vacuum drying and lyophilization techniques, the techniques can yield a powder of the active ingredient plus any additional desired ingredient present in the sterile-filtered solutions.

The compositions and preparations should contain at least about 0.1% of the active ingredient. The percentage of the compositions and preparations may, of course, be varied and may conveniently be between about 2-60% of the weight of a given unit dosage form. The amount of active ingredient in therapeutically useful pharmaceutical compositions is a level at which an effective dose can be obtained.

The amount of a compound described herein, or a pharmaceutically acceptable salt thereof, or other forms required for treatment may vary with the particular form selected, and may vary with the route of administration, the nature of the condition being treated and the age and condition of the patient, and may ultimately be at the discretion of the attendant physician or clinician.

A dose of a compound provided herein, or a pharmaceutically acceptable salt, or other forms, administered to a subject can range from 10µg~5,000mg; 10µg~1mg; 1~500mg; or 500-5,000 mg, etc. Preferably, convenient administration in unit dosage form. Each unit dosage form comprises, for example, 0.01~10mg or 0.05~1mg of active ingredient. In some embodiments, the dose is 5 mg/kg or less. The desired dose may be presented in a single dose or as divided doses administered at appropriate intervals.

Suitable dosages of the compounds described herein, or a pharmaceutically acceptable salt or other forms, can be determined by comparing the *in vitro* activity with *in vivo* activity thereof in animal models. Methods for extrapolating effective dosages in mice and other animals to humans are known in the art.

The phrases "physiologically acceptable carrier" and "pharmaceutically acceptable carrier", which may be used interchangeably, refer to a carrier or a diluent, including an adjuvant, that does not cause significant irritation to an organism and does not abrogate the biological activity and properties of the administered compound.

Exemplary solid carriers can include finely divided solids such as talc, clay, microcrystalline cellulose, silica, alumina and the like. Suitable liquid carriers include water, alcohols, glycols and water-alcohol/glycol blends. The compounds described herein, or pharmaceutically acceptable salts, or other forms, can be dissolved or dispersed at effective levels, optionally with the aid of non-toxic surfactants.

The term "excipient" herein refers to an inert substance added to a pharmaceutical composition to further facilitate administration of an active ingredient. Non-limiting examples of excipients include calcium carbonate, calcium phosphate, various sugars and various types of starch, cellulose derivatives, gelatin, vegetable oils and polyethylene glycols, and the like.

The present disclosure constructs a human neuronal model of JNCL disease by introducing homozygous deletion mutations in exons 7 and 8 of CLN3 gene, which is the most common in JNCL patients, into hESCs using CRISPR/Cas9 gene editing technology and inducing them to differentiate into neural lineages. This model recapitulates several JNCL-associated phenotypes, such as lysosomal and autophagy functional defects and dysregulation of mitochondrial homeostasis. Since the normal functions of autophagy and lysosomes are essential for maintaining neuronal homeostasis, high-throughput screening systems aimed at discovering novel autophagy-promoting small molecules have been established.

Through screening, a series of small molecule compounds that activate autophagy and lysosomal functions were obtained, and the effects of the compounds on alleviating the disease phenotype were tested in the constructed human neuron model of JNCL disease. The results showed that a small molecule, Compound G (guggulsterone), can effectively alleviate autophagy and lysosomal functional defects associated with JNCL disease and protect mitochondrial homeostasis in vitro. In the JNCL mouse model, guggulsterone had been proved to penetrate the blood-brain barrier and partially alleviate the learning and memory deficits and motor deficits exhibited by mice.

The mechanism of guggulsterone was further explored in combination with RNA sequencing and quantitative thiol reactivity analysis, it was found that guggulsterone could, through inhibiting KEAP1 protein, enhance autophagy and lysosomal function, and alleviate JNCL-accosicated disease phenotype.

### Experimental material

### Cell culture reagents:

Neurobasal^{®} medium, Advanced DMEM/F-12 medium, DMEM/F12 medium, DMEM basal medium, DPBS buffer, opti-MEM medium, fetal bovine serum (FBS), GlutaMAXTM (100×), N2 Supplement (100×), B27 Supplement (50×), Penicillin/Streptomycin (100×), Trypsin-EDTA, and StemPro Accutase were all purchased from Gibco; mTeSRTM1 medium was purchased from STEMCELL; borate buffer was purchased from Thermo Fisher Scientific; Matrigel was purchased from BD; Poly-D-lysine, PDL was purchased from Sigma Aldrich.

### Small molecules and cytokines:

CHIR99021 (S1263), SB431542 (S1067), rapamycin (S1039), Bafilomycin A1 (S1413), Y27632 2HCl (S1049) were purchased from Selleck; CAMP (A9501), L-ascorbic acid (A4403) were purchased from Sigma Aldrich; Compound G ((Z)-Guggulsterone) was purchased from Tocris; Human GDNF (450-10-500), Human/Murine/Rat BDNF (450-02-500) were purchased from PeproTech; Recombinant Mouse EGF Protein (2028-EG), Recombinant Mouse FGF basic (3139-FB) were purchased from R&D; Leukemia Inhibitory Factor Human (hLIF, LIF1010) was purchased from Millipore.

### Reagents for molecular biochemical experiment:

Lipofectamine^{®} 3000 Reagent, Hochest 33342, ECL developer and substrate luminescence solution (Super SignalTM West Dura Extended Duration Substrate), PageRuler^{™} Prestained Protein Ladder were purchased from Thermo Fisher Scientific; DMSO, paraformaldehyde, absolute ethanol, isopropanol, ammonium persulfate (AP), 30% Acr/Bis solution, tetramethylethylenediamine, sodium lauryl sulfate, iodoacetamide (IAA) were purchased from Sigma Aldrich; NEBuilder^{®} HiFi DNA Assembly kit was purchased from NEB; Proteinase K, Taq DNA polymerase, 6 × DNA Loading Buffer, 100bp DNA Ladder, Trans2K^{®} DNA Marker, PerfectStart^{™} Green qPCR SuperMix, EasyScript^{®} One-Step gDNA Removal and cDNA Synthesis SuperMix, competent cells were purchased from TransGen Biotech; Tris, glycine, sucrose were purchased from Amresco; anhydrous methanol was purchased from Beijing Tongguang Fine Chemical; protease inhibitors, phosphatase inhibitors were purchased from Roche; nitrocellulose membranes were purchased from Bio-Rad; PrimeSTAR^{®} Max DNA Polymerase was purchased from Takara; QuickBlock^{™} Western Blocking Solution, QuickBlock^{™} Western Primary Antibody Diluent, CellTiter-Lumi^{™} Steady Plus Luminescence Cell Viability Detection Kit and BCA Protein Assay Kit for concentration (P0010) were purchased from Beyotime; DTT (A620058-0025) was purchased from BBI Life Sciences; Light Az-UV-biotin (EVU102), Heavy Az-UV-biotin (EVU151) were purchased from KeraFast; DNeasy^{®} Blood & Tissue Kit (69506), QIAquick^{®} Gel Extraction Kit (28706), RNeazy Mini Plus Kit (74136), HiSpeed Plasmid Maxi Kit (12663) were purchased from QIAGEN; AxyPrep Total RNA Mini Prep Kit (AP-MN-MS-RNA) was purchased from Axygen; EndoFree Mini Plasmid Kit II (DP118-02) was purchased from Tiangen; Pierce^{™} Protein A/G Magnetic Beads (88802) was purchased from Thermo Fisher Scientific; Amaxa^{™} P3 Primary Cell 4D-Nucleofector^{™} X Kit L (V4XP-3024) was purchased from Lonza; Cathepsin D Activity Assay Kit (ab65302) was purchased from Abeam; Cathepsin-B Assay Kit (937) was purchased from ImmunoChemistry Technologies.

### Antibodies:

p62 antibody (PM045) was purchased from MBL; LC3 antibody (2775), β3-Tubulin antibody (5568P) were purchased from Cell Signaling Technology; β-Actin (C4) antibody (sc-47778) was purchased from Santa Cruz; Recombinant Anti-ATP synthase C antibody (ab181243), Pax6 antibody (ab195045) were purchased from Abeam; Sox1 antibody (AF3369) was purchased from R&D; β-actin antibody (sc-47778) was purchased from Santa Cruz; Nestin antibody (MAB353, MAB5326), Sox2 antibody (AB5603) were purchased from Millipore; MAP2 antibody (M9942) were purchased from Sigma Aldrich; Oct4 antibody (MA5-14845) was purchased from Thermo Fisher Scientific.

### Primer Sequence:

As shown in Table 1 below.

**Table 1: QPCR primer information for detecting gene expression**

| Gene | Upstream primer | Downstream primer |
|---|---|---|
| GAPDH | TGCACCACCAACTGCTTAGC | GGCATGGACTGTGGTCATGAG |
| CLN3 | GGTTGCCTTTTCTCATTCTGTG | CACCAGGAGATCACGGC |
| SOX2 | GTCATTTGCTGTGGGTGATG | AGAAAAACGAGGGAAATGGG |
| PAX6 | CCAGAAAGGATGCCTCATAAA | TCTGCGCGCCCCTAGTTA |
| NOGGIN | TCGAACACCCAGACCCTATC | CATGAAGCCTGGGTCGTAGT |
| SOX1 | CAATGCGGGGAGGAGAAGTC | CTCTGGACCAAACTGTGGCG |
| OCT4 | CAGTGCCCGAAACCCACAC | GGAGACCCAGCAGCCTCAAA |
| KEAP1 | CTGGAGGATCATACCAAGCAGG | GGATACCCTCAATGGACACCAC |
| HMOX1 | AAGACTGCGTTCCTGCTCAAC | AAAGCCCTACAGCAACTGTCG |
| NQO1 | GAAGAGCACTGATCGTACTGGC | GGATACTGAAAGTTCGCAGGG |
| TXNRD1 | TAGGACAAGCCCTGCAAGACT | CCCCAATTCAAAGAGCCAATGT |
| CTGF | CAGCATGGACGTTCGTCTG | AACCACGGTTTGGTCCTTGG |
| ATG5 | AAAGATGTGCTTCGAGATGTGT | CACTTTGTCAGTTACCAACGTCA |
| p62 | TACGACTTGTGTAGCGTCTG | CGTGTTTCACCTTCCGGAG |
| mtMinArc | CTAAATAGCCCACACGTTCCC | AGAGCTCCCGTGAGTGGTTA |
| b2M | GCTGGGTAGCTCTAAACAATGTAT TCA | CCATGTACTAACAAATGTCTAAAAT GGT |

### Plasmid:

pMXS-IP GFP-OMP25 (#38249), pBABE-puro-mCherry-EGFP-LC3B (#22418), pSpCas9(BB)-2A-Puro (PX459, #48139) were purchased from Addgene; ATG5 shRNA plasmid, KEAP1 shRNA plasmid, CLN3 shRNA plasmid were selected from MISSION^{®} shRNA plasmid library (MERCK); human p62-GFP plasmid (a plasmid that overexpresses p62 protein in mammalian cells driven by the efla promoter), Flag plasmid and Flag-KEAP1 plasmid (plasmids that overexpress FLAG and FLAG-KEAP1 proteins in mammalian cells, respectively, both driven by the CMV promoter).

### Experimental Animals:

Cln3 knockout mice B6.129S6-Cln3tm1Nbm/J (029471) was purchased from Jackson Laboratory and bred on a C57BL/6 background; wild-type C57BL/6 mice were purchased from Beijing Vital River Laboratory Animal Technology Co. Ltd. Animals were housed in independent ventilated cages with a maximum of 6 mice per cage. The mouse feeding environment had a 12/12 hour light/dark cycle and the ambient temperature was maintained at 22-26 °C. Mice had free access to sterile pelleted food and water. All animal experiments were approved by the Institutional Animal Care and Use Committee (IACUC).

### Cell culture and differentiation

### Human embryonic kidney cells HEK293T/17 cells:

HEK293T/17 cell medium (DMEM medium + 10% FBS + 1% Penicillin/Streptomycin) was used for adherent culture in a cell culture dish or cell culture plate with medium exchange daily, and it was passaged when the cell density reaches 95%-100%. During passage, the medium was aspirated and the cells were washed once with dPBS and then digested to single cells with 0.25% Trypsin-EDTA for 1 min at room temperature. HEK293T/17 cell culture was added with five times the volume of Trypsin-EDTA. Passage was performed at a ratio of 1:5 after resuspension by centrifugation.

### Rat kidney NRK cells:

NRK cell medium (NRK rat kidney cell medium: DMEM medium + 10% FBS + 1% Penicillin/Streptomycin) was used for adherent culture in a cell culture dish or cell culture plate with medium exchange daily, and it was passaged when the cell density reaches 95%-100%. During passage, the medium was aspirated and the cells were washed once with dPBS and then digested to single cells with 0.25% Trypsin-EDTA for 3 min at room temperature. NRK cell culture was added with five times the volume of Trypsin-EDTA. Passage was performed at a ratio of 1:10 after resuspension by centrifugation.

### Human embryonic stem cells:

H9 human embryonic stem cells were cultured on cell culture plates precoated with 2% matrigel. The medium was changed daily with fresh mTeSRTM1 medium. Passage was performed when the cell colony density reached 70%-80%. During passage, the medium was aspirated and the cells were washed once with dPBS before the addition of Versene and digestion at 37°C in a cell incubator for 5-7 min. After the cells had rounded but not yet floated, Versene was aspirated and the cells were blown into single cells using mTeSRTM1 medium with 10µM Y2763 and transferred to plates precoated with 2% matrigel at a ratio of 1:10-1:20. The medium on the day of passage was mTeSRTM1 medium with 10µM Y27632, which was removed after 24 hours. Wherein a human embryonic stem cell (hESC) medium: MTeSRTM 1 Medium + 1% Penicillin/Streptomycin.

### Differentiation and maintenance of human neural stem cells:

When the clonal density of human embryonic stem cells reached 80%, the medium was aspirated and the cells were washed once with dPBS before the addition of Versene and digestion at 37°C in a cell incubator for 5-7 min. After the cells had rounded but not yet floated, Versene was aspirated and the cells were blown into single cells using mTeSRTM1 medium with 10µM Y2763 and transferred to plates precoated with 2% matrigel at a ratio of 1:5. The medium on the day of passage was mTeSRTM1 medium with 10µM Y27632, which was changed to human neural stem cell induction medium 24 hours later (human neural stem cell induction medium: Advanced DMEM/F12 medium: Neurobasal^{®} medium (1:1) + 3µM CHIR99021 + 2µM SB431542 + 0.1µM Compound E + 5µg/mL BSA + 10ng/mL hLIF + 1×N2 + 1×B27 + 1% GlutaMAXTM + 1% Penicillin/Streptomycin), and continusly cultured for 7 days, to induce the formation of human neural stem cells, during which the medium was changed daily. 7 days later, the medium was aspirated and the cells were washed once with dPBS followed by the addition of Accutase and digestion at 37°Cin a cell culture incubator for 10 min. When the cells had rounded but not yet floated, Accutase was aspirated and the cells were blown into single cells using human neural stem cell maintenance medium (human neural stem cell maintenance medium: Advanced DMEM/F12 medium: Neurobasal^{®} medium (1:1) + 3µM CHIR99021 + 2µM SB431542 + 5µg/mL BSA + 10ng/mL hLIF + 1×N2 + 1×B27 + 1% GlutaMAXTM + 1% Penicillin/Streptomycin), then transferred to cell culture plates precoated 2% matrigel at a ratio of 1:3). Y27632 was removed after 24 hours. Thereafter, the cells were cultured in human neural stem cell and were passaged according to the above method when the cell density reached 85%-95%. Human neural stem cells can be maintained *in vitro* for more than 10 passages or can be stored in liquid nitrogen for later use.

### Differentiation and maintenance of human neurons:

When the density of human induced neural stem cells reached 85-95%, passage was performed at 1:20-1:50 according to the above method. The medium on the day of passage (recorded as Day 1 of differentiation) was human neural stem cell maintenance medium with 10µM Y27632, which was removed after 24h, and culture continued in human neural stem cell maintenance medium for 48 hours. On Day 4 of differentiation, the medium was changed to human neuronal differentiation medium (human neuronal medium: DMEM/F12 medium + 10ng/ml hBDNF + 10ng/ml hGDNF + 300ng/mL cAMP + 0.2mM vitamin C + 1×N2 + 1×B27 + 1% Penicillin/Streptomycin), with medium change halfly every other day. Human neurons highly expressing MAP2 and TUJ1 proteins were obtained on Day 17 of differentiation. Thereafter, the human neurons could be continuously cultured in the human neuron differentiation medium, with medium change halfly every other day.

### Flow cytometry analysis and sorting:

In analyzing the mean fluorescence intensity of neural stem cells and neuronal staining or single cell establishment of NRK cells, flow cytometry was used: the cell medium was aspirated off, after wash with dPBS once, the digestive enzyme corresponding to the cell type was added to digest into single cells; the supernatant was removed by centrifugation, and the cells were resuspended with 1mL of fresh medium; the cell suspension with filtered through 40µm filter membrane and the single cell filtrate was collected; the single cell filtrate was transferred to a flow tube and analyzed or sorted using BD FACS Aria III; in the analysis, the fluorescence intensity of 1×10⁴ cells for each sample was analyzed to obtain the mean fluorescence intensity (MFI); for sorting, cells were received in 96 well plates with medium, one cell per well to establish a cell line.

### Cell transfection

### Cell electroporation:

For CRISPR/Cas gene editing of human embryonic stem cells, transfection was performed with Amaxa^{™} P3 primary cell 4D Nucleofector^{™} electrotransfection kit, taking 12-well cell culture plate as an example: the Amaxa^{™} P3 primary cell 4D Nucleofector^{™} electrotransfection kit was equilibrated to room temperature in advance; human embryonic stem cells were digested with versene at 37°C for 10 minutes after growing to a density of about 70%; cells were blown into single cells with 1mL of mTeSRTM1 medium with 10µM Y27632; centrifugation at the rotation speed of 500g for 5 minutes with a desk centrifuge to remove the supernatant as clean as possible; 82µL Nucleofector^{™} Solution and 18µL Supplement contained in the kit were mixed, and PX459 plasmid and two sgRNA plasmids targeting *CLN3* were added at 1µg, respectively, followed by mixing with blow. Said human embryonic stem cells were resuspended with the obtained solution and transferred to an electrotransfection cup, and bubble generation was avoided duting the transfer process; electrotransformation was performed with Lonza electrotron, and the electrotransformation program is CB-150; the electroporated cells were transferred into 5mL of fresh mTeSRTM1 medium comprising 10µM Y27632 and seeded into cell culture plates precoated with 2% Matrigel; after 24 hours of electroporation, Y27632 was removed and switched to fresh mTeSRTM1 medium, and the electroporation was completed.

### Liposome 3000 cell transfection:

HEK293T/17 cells used for co-immunoprecipitation were mainly transfected by Lipofectamine 3000 kit. Taking 10cm cell culture dish as an example: HEK293T/17 cells were cultured to 40-50% density; firstly, 10µg of plasmid and 20µL of P3000 reagent were added into 500µL of opti-MEM medium and mixed by shaking; 30µL of Liposome 3000 was added into 500µL of opti-MEM medium in another tube and mixed by reversal; Liposome 3000 was added into the above opti-MEM medium containing the plasmid and mixed by reversal; after leaving at room temperature for 15 minutes, it was dropwised to the HEK293T/17 cell plate. 8 hours after transfection, the cell medium was aspirated and fresh HEK293T/17 cell medium was added, and the transfection completed; 36 hours after transfection, cells can be collected for co-immunoprecipitation experiments.

### Lentivirus packaging and infection

Lentivirus packaging was performed in HEK293T/17 cells: HEK293T/17 cells were plated in cell culture plates, until a density of 40-60%; the lentivirus plasmid and two viral packaging plasmids psPAX2 and pMD2.G were mixed in a ratio of 5:3:2 for transfection, and the transfection step referred to the transfection step of liposome 3000 cells; after 8 hours of transfection, liposome 3000 was aspirated and replaced with fresh HEK293T/17 cell medium; 32-56 hours after transfection, the HEK293T/17 cell medium was collected and the cell debris was filtered off with a 0.45µM filter; according to the required amount of virus, the filtered virus solution was added into the medium of target cells, and the remaining virus can be quickly frozen in liquid nitrogen and stored at -80°C for subsequent use, and repeated freezing and thawing should be avoided; 8 hours after infection the medium was replaced with fresh medium of the target cells, and the infection completed.

### Western Blot

Sample preparation: for adherent cultured cells, exemplified by cells cultured in a six-well cell plate, the cell medium was aspirated and the cells were washed once with dPBS. About 100uL of RIPA lysate (50 mM Tris-HCl, pH 7.5, 150 mM NaCl, 0.25% Na-deoxycholate, 0.1% NP-40, 0.1% Triton X-100) containing protease inhibitors and phosphatase inhibitors was added to each well. The cell lysate was scraped off completely with a cell scraper and collected into 1.5mL EP tubes. Lysis was continued for 30 minutes on ice. For animal tissue, the weight of the animal tissue was weighed, and RIPA lysate containing protease inhibitors and phosphatase inhibitors was added with five times the volume of the animal tissue. The tissue was grinded on ice with a tissue grinder until there is no obvious tissue mass, collected into 1.5mL EP tubes, and placed on ice for further lysis for 30 min.

The cell or tissue lysate was centrifuged at 13,000 rpm at 4°C for 5 minutes, and the supernatant was collected; the protein concentration in the lysis solution was determined using BCA Protein Assay Kit for concentration, and the sample was diluted to the target concentration; 4 volumes of lysate supernatant was mixed with 1 volume of 5× loading buffer (0.3125M Tris-HCl, pH 6.8, 10% SDS, 50% glycerol, 0.25% bromophenol blue, 0.5M DTT) and heated at 98°C for 10 minutes; polyacrylamide gel electrophoresis: the protein samples were electrophoresed by SDS-PAGE (7.5% concentration gel + 10% separation gel for p62, CLN3; 7.5% concentration gum + 15% separation gum for LC3, SCMAS); the composition of electrophoresis buffer is 25 mM Tris-Base, 0.2 M glycine, 1% SDS, pH 8.3; the electrophoresis conditions were 80 V for 30 minutes and 120 V for 1 hour; transmembrane: proteins were transferred from the separation gel to PVDF membranes by wet transfer (0.45µM membrane for p62, CLN3; 0.22µM membrane for LC3, SCMAS); transmembrane solution formulation is 25 mM Tris-Base, 0.2 M glycine, 20% methanol, pre-cooled to 4°C; transmembrane conditions are 150 mA for 1-2 hours; blocking: protein-transferred PVDF membrane was placed in QuickBlock^{™} Western blocking solution, blocked for 1 hour at room temperature on a shaker, and washed with PBST for 3 times for 5 minutes each; primary antibody incubation: antibody was diluted to QuickBlock^{™} Western primary antibody diluent according to the proportion indicated in the specification, then added to the blocked PVDF membrane, incubated on a shaker at 4°C overnight or at room temperature for 2 hours, and washed with PBST for 3 times for 5 minutes each; secondary antibody incubation: the secondary antibody of the corresponding species was diluted with PBST at a ratio of 1:5000, then incubated on a shaker at room temperature for 1 hour, and washed with PBST 3 times for 5 minutes each; color development: the ECL chromogenic solution substrate and the luminescent solution were mixed in a ratio of 1:1 then dropped onto a PVDF membrane, and color development was performed using a chemiluminescence imager after reaction at room temperature for 5 minutes; band grayscale calculation: the grayscale of the target protein was quantitatively analyzed by ImageJ software.

### Immunofluorescence staining

Fixation: the cell medium was aspirated and the cells were washed once with dPBS, then 4% paraformaldehyde solution was added to fix the cells, which were then incubated at room temperature for 20 min and washed 3 times with dPBS for 5 min each; blocking and permeabilization: blocking solution (5% BSA and 0.3% Trinton-X100 dissolved in dPBS) was added, incubation at room temperature for 1 hour followed by washing with dPBS 3 times for 5 minutes each; primary antibody incubation: the antibody was diluted with dPBS containing 5% BSA according to the specification, incubated overnight at 4°C or 2 hours at room temperature, and washed 3 times with dPBS for 5 minutes each; secondary antibody incubation: the secondary antibody of the corresponding species was diluted with dPBS containing 5% BSA at a ratio of 1:1000, incubated at room temperature for 1 hour, and then washed with dPBS 3 times for 5 minutes each; nuclear staining: DAPI was diluted to 100ng/mL with dPBS, incubated at room temperature for 5 minutes, and washed 3 times with dPBS for 5 minutes each; imaging: imaging was performed using Olympus IX83 Inverted Motorized Microscope or Opera Phenix^{™} High Content Screening System.

### Immunohistochemical staining

Immunohistochemical staining of mouse brain tissue: the mice were anesthetized by intraperitoneal injection of Avertin anesthetic, and following thoracotomy, the heart was perfused with 20mL of pre-cooled dPBS to wash out the blood in the miceand them was perfused with 20mL of pre-cooled 4% paraformaldehyde solution to pre-fix the tissues; the complete mouse brain tissue was removed through dissection and placed in a 50mL centrifuge tube containing 4% paraformaldehyde solution, fixing at 4°C overnight; 4% paraformaldehyde solution was discarded, wash was performed 3 times with dPBS; dPBS solution containing 15% sucrose was added, dehydrated at 4°C for 12h until brain tissue sank to the bottom of 50mL centrifuge tube; dPBS solution containing 15% sucrose was discarded and dPBS solution containing 30% sucrose was added, dehydrated at 4°C for 12h to 24h until brain tissue sank to the bottom of 50mL centrifuge tube; the brain tissue was taken out, and the surface moisture of the tissue was dried with absorbent paper, then the brain tissue was wrapped with OCT embedding agent and frozen at -20°C; the brain tissue was sliced to slices with a thickness of 30 µm by a freezing microtome, and the sliced brain slices were washed 3 times in dPBS; 4% paraformaldehyde solution was added to fix the cells, incubated at room temperature for 20 minutes, and then washed three times with dPBS for 5 minutes each time.

blocking and permeabilization: blocking solution (5% BSA and 0.3% Trinton-X100 dissolved in dPBS) was added, incubation at room temperature for 1 hour followed by washing with dPBS 3 times for 5 minutes each; primary antibody incubation: the antibody was diluted with dPBS containing 5% BSA according to the specification, incubated overnight at 4°C, then washed 3 times with dPBS for 5 minutes each; secondary antibody incubation: the secondary antibody of the corresponding species was diluted with dPBS containing 5% BSA at a ratio of 1:1000, incubated at room temperature for 1 hour, and then washed with dPBS 3 times for 5 minutes each; nuclear staining: DAPI was diluted to 100ng/mL with dPBS, incubated at room temperature for 5 minutes, and washed 3 times with dPBS for 5 minutes each; the the brain tissue was plated on a glass slide, an anti-quenching agent was dripped, then a cover glass was covered, the surrounding area of the cover slip was sealed with transparent nail polish, and air-dried at room temperature in the dark; imaging: imaging was performed using Olympus IX83 Inverted Motorized Microscope or Zeiss LSM 780.

### Determination of intracellular relative ATP content

Neural stem cells or neurons were digested into single cells and were counted using a hemocytometer. The cell suspension was then diluted to the same cell concentration (10^{5~}10⁶/mL). 100 µL of cell suspension was taken out into a white 96-well analysis plate, and 100 µL of CellTiter-Lumi^{™} Steady Plus reagent that had been equilibrated to room temperature was added, shaken to mix, and incubated at room temperature for 15 minutes. The chemiluminescence intensity of the sample was then read using a multifunctional microplate reader to indicate the intracellular relative ATP content.

### Determination of intracellular relative mitochondrial copy number

The medium in the cell culture plate was aspirated and the cells were digested after washing once with dPBS. After stopping the digestion with cell medium, the supernatant was removed by centrifugation and the cells were resuspended with 200µL dPBS. DNeasy^{®} Blood & Tissue Kit was used to extract DNA from cells. Then, referring to the qRT-PCR system and reaction procedure in Tables 2 and 3, the qRT-PCR reaction was performed using the extracted DNA instead of the cDNA. Data analysis was performed in Prism 7 software using the 2^{-ΔΔCt} process with genomic gene b2M as internal reference for relative quantification of the number of mitochondrial sequences MinArc. Primers for determining intracellular mitochondria number were as follows:
*mtMinArc-F*: CTAAATAGCCCACACGTTCCC;
*mtMinArc-R*: AGAGCTCCCGTGAGTGGTTA;

Primers for determining the intracellular genome number were as follows:
*b2M-F:* GCTGGGTAGCTCTAAACAATGTATTCA;
*b2M-R:* CCATGTACTAACAAATGTCTAAAATGGT.

### RNA extraction and Real-Time Quantitative Reverse Transcription PCR (qRT-PCR)

The cells in the cell-culture dish were collected, extracted using AxyPrep Total RNA Mini Prep Kit or Qiagen AxyPrep Total RNA Mini Prep Kit, and the extracted RNA concentration is measured using Nanodrop; the extracted mRNA was reverse transcribed into cDNA by Transgen's EasyScript^{®} One-Step gDNA Removal and cDNA Synthesis SuperMix and the primers used for reverse transcription were Oligo dT primers; the completion of reverse transcription followed by qRT-PCR reaction through PerfectStart^{™} Green qPCR SuperMix to detect the expression of the target gene; the qRT-PCR reaction was performed on the Bio-Rad CFX96 Real-Time PCR system; see Table 2 and Table 3 for the 20µL qRT-PCR reaction system and reaction procedures; data analysis was performed in Prism 7 software using the 2^{-ΔΔCt} process with GAPDH as internal reference for relative quantification of the expression of the target gene.

**Table 2: qRT-PCR Reaction System**

| Component | Volume (µl) |
|---|---|
| PerfectStart^{™} Green qPCR SuperMix | 10 |
| Forward primer (10 pmol) | 1 |
| Reverse primer (10 pmol) | 1 |
| cDNA | X |
| dd H₂O | 12-X |
| Total volume | 20 |

**Table 3: qRT-PCR Reaction Procedures**

| Step | Temperature | Time/Cycle | Cycle Number |
|---|---|---|---|
| Thermal activation of DNA polymerase and template denaturation | 94°C | 3 minutes | 1 |
| Denaturation | 94°C | 10 seconds | |
| Annealing/Extension | 60°C | 30 seconds | 40 |
| Melt Curve | Ramp from 55°C to 95°C, increasing by 0.5°C per cycle, 5 seconds per cycle | | |

### Determination of intracellular Cathepsin D activity

The activity of intracellular protease cathepsin D was measured using Abcam's Cathepsin D Activity Assay kit. Each sample was prepared in more than 3 replicates. The specific steps were as follows: the medium in the cell culture plate was aspirated and the cells were digested into single cell and resuspended in fresh cell medium after washing once with dPBS; a blood cell counting plate was used to count the cells, for each sample, 10⁶ cells were taken and washed once with 100µL dPBS after centrifuging to remove the supernatant, and centrifuged at 4°C to remove the supernatant; 200µL of CD cell lysate contained in the kit was added followed by incubation on ice for 10 minutes; centrifugation at 13000 rpm for 5 minutes at 4°C and the supernatant was collected into a new 1.5mL EP tube; 50µL reaction solution was mixed with 2µL substrate, 40µL CD lysis buffer and 10µL cell supernatant from step 4) were added, transferred to a cell culture plate with a black wall and a transparent bottom, and incubated at 37°C in the dark for 1-2 hours; the fluorescence intensity of the sample was read using a multifunctional microplate reader, and the excitation and emission wavelengths were 328nm and 460nm respectively; the ratio of the fluorescence intensity between the experimental group and the control group is the relative fold change of cathepsin D enzyme activity in the cells of the experimental group.

### Determination of drug concentration in tissue

In order to determine whether Compound G can pass through the blood-brain barrier and its drug concentration in the brain tissue of mice, the following experiment was performed: Compound G was dissolved in drinking water at a concentration of 1 mg/mL and fed to mice, and a solvent was added to the drinking water of control mice; mice treated with Compound G were removed at 8 different random time points, and after Avertin anesthesia, their hearts were perfused with 20mL dPBS to rinse the blood in the mice; non-dosed mice were also perfused in the same manner as blank tissue; whole mouse brain tissue was removed, weighed, snap frozen in liquid nitrogen and ground using Mixer Mill (RETSCH MM400) with a shaking frequency of 26 beats/sec for 5 minutes; a small molecule with similar structural properties to Compound G was prepared in advance as an internal reference (denoted IS) and IS was dissolved in 80% aqueous methanol to 100ng/mL.

According to the mouse brain tissue tumor before grinding, the IS methanol solution in step 4) was added to the ground brain tissue in a volume of 5mL per gram of brain tissue and was fully mixed by shaking; the supernatant was collected after centrifuging at 13000 g for 8 minutes at 4°C; 100 µL of the supernatant was mixed with 100 µL of 10% aqueous methanol and transferred to an HPLC sample vial; the supernatant of blank tissue was mixed with 100 µL of 10% aqueous methanol and, and the the standard of Compound G was added to the following concentration nd transferred to HPLC sample vial as standard curve: 1000ng/mL, 500ng/mL, 200 ng/mL, 100ng/mL, 50ng/mL, 20ng/mL, 10ng/mL, 5ng/mL, 2ng/mL, 1ng/mL, 0.5ng/mL, 0.2ng/mL, 0.1ng/mL; UPLC-MS/MS was used to identify the peak area of Compound G in the sample.

The chromatographic conditions were as follows: the chromatographic column was Waters XSelect HSS T3 column (100×2.1mm, 1.8µm); mobile phase A was 0.1% formic acid methanol, mobile phase B was 0.1% formic acid water; gradient elution: 40% mobile phase A to 100% mobile phase A over 0-3 minutes, then a hold for 2 minutes, then returned to the initial 40% mobile phase A at Minute 5.1, and ended one run at Minute 8; the flow rate was 0.2mL/min; the injection volume was 10µL; the column temperature was 30°C.

The mass spectrometry conditions were as follows: electrospray ionization (ESI), in positive ion mode, multiple reaction monitor (MRM), with an ion source temperature of 500°C, curtain gas of 30 psi, collision gas of medium, ion voltage of 5500 V, spray gas and auxiliary heating gas of 50 psi; the peak area of Compound G was calculated by AB SCIEX Analyst 1.6.3 Software and a standard curve was formed with standards, to calculate the drug concentration in the sample.

**Table 4: Mass spectrometry parameters**

| Sample | Q1-Q3 | DP | CE |
|---|---|---|---|
| Compund G | 313.1/97* | 170 | 27 |
| Compund G | 313.1/109 | 170 | 24 |
| IS | 361.1/343.3* | 105 | 14 |
| IS | 361.1/147.1 | 105 | 28 |

| | | | |
|---|---|---|---|
| The maker * indicates quantitative fragment ion. | | | |

### Quantitative Thiol Reactivity Analysis

To determine the protein covalently bound to Compound G, a Compound G-binding proteomic analysis with the following steps was performed: neural stem cells were cultured in 10cm cell culture dishes to a cell density of 90%. 1-2 mg of protein could be obtained after the neural stem cells in a 10 cm cell culture dish are lysed; the supernatant was aspirated, fresh medium containing 30µM Compound G or medium containing an equal volume of solvent DMSO was added, followed by incubation in a cell culture incubator at 37°C for 2 hours; the cell medium was aspirated followed by washing once with 10mL of dPBS, and the aspiration should be as clean as possible.

The cell culture dish was placed on ice and added with RIPA cell lysis buffer with protease inhibitors and phosphatase inhibitors (for the formulation, please refer to the flow cytometry analysis and sorting section); all cells were scraped off on ice using a cell scraper and collected into 1.5mL EP tubes; the sample was placed on ice and ultrasonically broken with an ultrasonic power of 20% and with ultrasound 5 times for 3 seconds each time; ENE probe at a final concentration of 100 µM was added to each sample, followed by incubation for 1 hour in the dark while rotating. ENE could react with sulfhydryl groups in protein cysteine residues to produce stable thioether bonds.

The reaction was stopped by the addition of ENE probe at a final concentration of 100µM to each sample, followed by incubation for 1 hour in the dark while rotating; each sample was added with freshly prepared iodoacetamide (IAA) at a final concentration of 32mM and incubated in the dark with rotation for 0.5 hours to alkylate the reduced cysteine; each sample was added with 500 µL of pre-cooled anhydrous methanol and 125µL of pre-cooled chloroform and mixed by inversion, followed by centrifugation at 1700g for 30 minutes at 4°C to remove the supernatant; each sample was added with 500µL of pre-cooled anhydrous methanol and 500µL of pre-cooled chloroform and mixed by inversion, followed by centrifugation at 1700g for 30 minutes at 4°C to remove the supernatant; repeated.

The sample was heated slightly in a 37°C metal bath to evaporate the residual liquid, with the EP tube lid opened; 200µL of trypsin digestion buffer containing 1M urea (4 mg/ml ammonium bicarbonate in water, pH=8) was added, and the protein precipitate was sonicated on ice; 200µL trypsin digestion buffer without urea was added, and the protein was dissolved by ultrasonic at 20% power, 10 seconds each time, 5 times, until no protein particles were visible to the naked eye; protein quantification was performed using the BCA Protein Assay Kit for concentration; the protein concentration was diluted to 1 mg/mL by trypsin digestion buffer, and 1mL of the diluted protein was transferred to a new 1.5 mL EP tube; 20 µg Sequencing Grade Trypsin stock solution (Promega, V5113) was added, followed by incubation overnight at 37°C in a hot stirrer; centrifugation at 14000g for 5 minutes at room temperature, and the supernatant was collected into a new 1.5mL EP tube; the sample was concentrated to about 200µL by vacuum centrifugation; desalting treatment was carried out using HLB SPE filter element, which could effectively separate peptides and inorganic salts under neutral conditions.

1 mL of HPLC grade acetonitrile was added to the HLB SPE filter, allowing it to flow through the filter through gravity; 1 mL HPLC grade water was added to equilibrate the filter; repeated. About 200µL of sample was added to the adsorption bed of the filter element; 1mL of HPLC grade water was added to clean the filter element; 1mL of HLB solvent A (formula: 80% acetonitrile, 10% methanol, and 10% HPLC grade water) was added to the filter element, and the eluate containing peptides was collected. The collected peptides were centrifuged using a vacuum centrifuge at 45°C for about 2 hours to evaporate to dryness; 20µL HPLC grade water and 10µL acetonitrile were added to the peptide fragments being evaporated to dryness, followed by reconstitution through shaking; L Az-UV-biotin (samples treated with Compound G) or H Az-UV-biotin (control treated with DMSO) with a final concentration of 1mM, 4mM sodium ascorbate, 1.25mM TBTA and 4mM copper sulfate were added in sequence and shaken gently to mix, followed by incubation at room temperature in the dark for 2 hours; 120µL streptomycin binding buffer (50mM NaOAc, pH 4.5) was added to dilute the reaction mixture, and immediately vortexed for 30 seconds to mix; centrifugation at room temperature for five minutes at 14,000g, and equal amounts of the supernatant of the Compound G-treated sample and the control sample were taken out and mixed in a new 1.5mL EP tube; 200 µL of streptomycin beads were transferred to a 15 mL tube and washed twice with 10 mL of streptomycin binding buffer; 10 mL of streptomycin binding buffer was added to resuspend the streptomycin beads, and the mixed sample in step 29 was added, followed by incubation at room temperature in the dark for 2 hours; centrifugation at room temperature for 3 minutes at 1700 g to remove the supernatant; the streptomycin beads were washed twice each with streptomycin binding buffer, streptomycin washing buffer (50mM NaOAc, 2 M NaCl, pH 4.5) and HPLC grade water; the streptomycin beads were resuspended in 1.2 mL of photolysis buffer (25 mM NH₄HCO₃) and transferred to a thin-walled glass vial with a magnetic stir bar; the glass bottle was placed on a magnetic stirrer and irradiated with 365nm UV rays using a handheld UV irradiation lamp at room temperature for 2 hours; the streptomycin bead suspension was transferred to a new 2 mL EP tube and centrifuged at 2000 g for 4 min at room temperature. The supernatant was collected and transferred to a new 1.5mL EP tube; then concentrated to approximately 200µL using a vacuum centrifuge. Final desalination was performed with C18 StageTips filters. 1 mL of HPLC grade acetonitrile was added to the filter, allowing it to flow through the filter through gravity; 1 mL HPLC grade water was added to equilibrate the filter; repeated. The above concentrated liquid was added to the adsorption bed of the filter element, which was then washed with 1mL HPLC grade water; the peptides were eluted with 750 µL HLB solvent A and 250 µL HLB solvent B, and the eluates were mixed in a new EP tube.

The eluted peptide sample was dried with a vacuum centrifuge at 45°C, which took about 2 hours; 12µL of LC-MS/MS sampling buffer (0.1% formic acid and 5% acetonitrile in water) was added to resuspend the peptides; centrifugation at 20,000g for 10 minutes at room temperature, and 10 µL of the supernatant was transferred to a sampling bottle with a cannula; the sampling bottle was placed into the LC autosampler pre-cooled to 4°C, and the injection program was set and started. For each analysis, half (5 µL) of each sample was injected into the LC-MS/MS and the flow rate was set to 600 nL/min; LC-MS/MS results were searched and analyzed using pFind 3.

### Co-immunoprecipitation

Flag plasmid and Flag-KEAP1 plasmid were transfected into cells using Lipofectamine 3000; 36-48 hours after transfection, the cell culture medium was removed and the cells were washed once with pre-chilled dPBS; taking a 10cm cell culture dish as an example, PBS was aspirated, and 1 ml of pre-cooled non-denaturing cell lysis solution containing protease inhibitors and phosphatase inhibitors (2mM Tris•HCl (pH 7.4), 150mM NaCl, 1% NP-40, 1mM EDTA, 5% glycerol) was added, followed by incubation on ice for 5 minutes; cells were scraped off on ice using a cell scraper, collected into 1.5mL EP tubes, and placed on ice; the sample could be sonicated 3 times for 5 seconds on ice; centrifugation at 14,000 g for 10 minutes at 4°C, then the supernatant was transferred to a new tube, which was placed on ice; the supernatant was allowed for long-time storage at -80°C; protein quantification was performed using the BCA Protein Assay Kit for concentration, followed by dilution to 1mg/ml; the protein A/G magnetic beads were gently vortexed to resuspend, 20 µL of the bead slurry was transferred to a 1.5 mL EP tube, which was placed on the magnetic separation rack for 10-15 seconds; the solution is carefully aspirated after the solution becoming clear; 500 µL of non-denaturing cell lysis buffer was added, followed by washing twice.

200 µL of diluted cell lysate was added to the magnetic beads, followed by incubation with rotation at room temperature for 20 minutes; the cell lysate and magnetic beads were separated using a magnetic stand, and the cell lysate was transferred to a new 1.5mL EP tube; 2.5µg Flag antibody or IgG isotype control of the same species was added to the cell lysis solution and incubated overnight at 4°C with rotation; magnetic beads were washed; the cell lysate and antibody are transferred to washed magnetic beads, followed by incubation with rotation at room temperature for 20 minutes; the cell lysate and magnetic beads were separated using a magnetic stand, and the clarified solution was aspirated; the magnetic beads were washed 5 times with non-denaturing cell lysis solution containing protease inhibitors and phosphatase inhibitors, and during washing, the samples were kept on ice; the cell lysate and magnetic beads were separated using a magnetic stand, and the supernatant was aspirated as much as possible, then 32 µL of non-denaturing cell lysate and 8 µL of 5× loading buffer were added, and heated at 98°C for 10 minutes to elute the target protein from the magnetic beads; western blotting was performed as described above.

### Treatment of Cln3 KO mice with exemplary Compound G

To compare the differences between *CIn3 KO* mice and wild-type mice, and the therapeutic effect of exemplary compound G on *CIn3 KO* mice, 21-Day-old mice were isolated from breeding cages and treated with continuous addition of 1 mg/mL Compound G in their drinking water. The control group was *Cln3 KO* mice whose drinking water contained an equal amount of solvent. Body weight changes were recorded during administration. At the age of Month 2, the mice were euthanized and immunohistochemical staining of brain tissue was performed to observe the abnormal accumulation of SCMAS proteins in the brain. At the age of Month 4-5, the differences in learning and memory abilities and motor abilities as well as the therapeutic effect of Compound G on *Cln3 KO* mice between *Cln3 KO* mice and wild-type mice were compared by Morris water maze, rotarod test and open field test.

### Morris water maze

To test the difference in learning and memory abilities of *Cln3 KO* mice and wild-type mice, and the effect of Compound G on learning and memory abilities of *Cln3 KO* mice, a Morris water maze was performed, with specific procedures as follows: the mice to be tested were kept in single cages for more than 1 week to adapt to the environment; the maze used in this study was a circular pool with a diameter of 120cm and a depth of 40cm; the pool wall is divided into four quadrants including northeast (NE), northwest (NW), southwest (SW) and southeast (SE) with circles, squares, triangles and crosses respectively. The platform was placed at a fixed position in the SW quadrant with a diameter of 11 cm and a height of 18 cm; milky white water containing titanium dioxide was added to the pool, the amount of added water being just short of the platform is suitable; the water temperature was adjusted to about 22°C; the mouse was gently put into the water from the designated position shown in Table 5, and the movement of the mouse within 60 seconds was recorded through the video equipment. If the mouse found the platform within 60 seconds, its time to find the platform was recorded; if the mouse could not find the platform within 60 seconds, it was guided to the platform and held for 15 seconds.

The mice were removed from the platform, wiped clean with a dry towel and returned to their cages. Each mouse was trained 4 times a day, with a 30-minute interval between each training, for a total of 6 days, and mice that cannot swim were excluded; on the Day 7, the platform in the water was taken out, and then the mice were gently put into the water from the NE direction, and the movement of the mouse within 60 seconds was recorded with a video equipment. The time it took for the mouse to find the platform for the first time and the time the mouse stayed in the SW quadrant were analyzed and counted using Noldus software. In addition, the experimenter manually counted the number of times the mouse crossed the location of the original platform within 60 seconds.

**Table 5: Location information of mice placed in the water maze**

| Training days | 1^{st} time | 2^{nd} time | 3^{rd} time | 4^{th} time |
|---|---|---|---|---|
| Day 1 | N | E | SE | NW |
| Day 2 | E | SE | NW | N |
| Day 3 | SE | NW | N | E |
| Day 4 | NW | N | E | SE |
| Day 5 | N | E | SE | NW |
| Day 6 | E | SE | NW | N |
| Day 7 (Probe) | NE | | | |

### Rotarod Test

To test the difference in exercise ability between *Cln3 KO* mice and wild-type mice, as well as the effect of Compound G on the exercise ability of *Cln3 KO* mice, an open field test was conducted with specific steps as follows: the mice to be tested were kept in single cages for more than 1 week to adapt to the environment; the rotation speed of the wheel was set to 10 rpm, and the mice were gently placed on the device to adapt for 1 minute; the rotation speed was increased continuously and reached 30 rpm within 300 seconds, and if the mice fell from the roller during this period, the device senced and recorded the time of the mice's first falling, thereafter, the dropped mice were put back into the device for continue training; if the mice did not fall during the period, it was recorded as 300 seconds; the mice was returned to the cage if falling more than 3 times within 300 seconds; the mice were trained three times a day, with an interval of 30 minutes each time, and the training lasted for a total of 4 days; on Day 4, the average value of the three training results could be used as an indicator to measure the mouse's exercise ability.

### Open Field Test

Open field tests were conducted on mice using blue boxes measuring 50 cm × 50 cm × 40 cm, with specific steps as follows: the mice to be tested were kept in single cages for more than 1 week to adapt to the environment; the central 25 cm × 25 cm area of the box was divided into a central area. The mice were placed gently in the central area facing away from the experimenter, and the video device was started to record the movement track of the mice within 10 minutes; the boxes were thoroughly wiped twice with 75% ethanol to eliminate the odor of the mouse, and then the next mouse was placed for movement track record within 10 minutes; the movement track of mice was analyzed with the Noldus software, and the analysis indicators included the movement speed and total movement distance of mice. The number of times the mice stood upright on their hind legs within 10 minutes was manually counted by the experimenter.

### Lentivirus-Mediated Target Protein Knockdown

shRNA knockdown experiments of genes such as *CLN3* and *KEAP1* were conducted in induced neural stem cells and neurons, with specific steps as follows: the neural stem cells were planted into a desired cell culture plate to a cell density of about 50% or the neural stem cells were differentiated into human neurons in the cell culture plate according to experimental requirements; the shRNA lentiviral plasmids of *CLN3* and *KEAP1* genes and the control lentiviral plasmid expressing non-target sequences were selected from the MISSIOH human shRNA library of Sigma Aldrich, and the sequences were shown in Table 6; Lentiviruses was packaged in 10 cm cell culture plates and virus-containing supernatant was collected by filtration according to the method described above; the supernatant and lentivirus concentrate (40% PEG-8000 and 1.2M sodium chloride dissolved in dPBS solution, pH ~ 7.0) were mixed in a volume ratio of 1:3, and incubated overnight at 4°C with slow rotation; centrifugation at 13000 g for 30 minutes at 4°C, and the supernatant was discarded, and the pellet was resuspended in 1 mL of fresh cell culture medium and added to the target cells; after 8 hours of infection, the virus was aspirated and fresh cell culture medium was added; 48 hours after infection, 1.5 µg/mL puromycin was added for selection for 72 hours, and the surviving cells were used for subsequent experiments.

**Table 6: shRNA sequences for gene knockdown**

| **Name** | **shRNA Sequence** |
|---|---|
| *CLN3* | CCGGCGTAGTTTACTTTGCCGAGTACTCGAGTACTCGGCAAAGTAAA CTACGTTTTTG |
| *KEAP1* | CCGGGCGAATGATCACAGCAATGAACTCGAGTTCATTGCTGTGATC ATTCGCTTTTTG |

### Transcriptome Sequencing Test

In order to detect the effect of Compound G on treated cells, transcriptome sequencing was performed on neural stem cells treated with Compound G, with specific steps as follows: *CLN3*^{*Δex7*/*8*} human neural stem cells were seeded into 2% Matrigel-coated 6-well cell plates to a cell density of 80%; fresh cell culture medium containing 6 µM Compound G or an equal volume of DMSO was added, and each treatment condition for two biological replicates; the neural stem cells were incubated in a 37°C cell culture incubator for 6 hours; the cell culture medium was aspirated, following by washing once with dPBS, and then intracellular RNA was extracted by Qiagen RNeazy Mini Plus Kit; the extracted RNA was sent to ANNOROAD for RNA quality testing first. The purity of RNA was tested using a Nanophotometer spectrophotometer; the concentration of RNA was tested using a Qubit 3.0 Flurometer; the integrity of the RNA was tested using the Aligent 2100 RNA Nano 6000 Assay kit; 2 µg of RNA was taken from each qualified sample for library construction and quantified using Qubit 3.0 Flurometer.

After diluting the library-building samples to 1ng/µL, the Aligent 2100 RNA Nano 6000 Assay kit and qPCR were used for quality detection; Illumina's Hiseq PE Cluster kit was used for clustering, and the Hiseq 2500 sequencing platform was used for paired-end sequencing to obtain 150 bp paired-end sequencing reads; FastQC software was used to evaluate the quality of the raw data obtained from sequencing, then TrimGalore was used to remove adapter sequences and low-quality sequences, and filtered data was obtained for subsequent analysis.

Comparation between filtered data and human reference genome hg38 was carried with HISAT2, resulting in Barn file, and the number of counts aligned to each gene was counted by FeatureCount, with gtf (GRCh38.92) file provided by Ensembl as annotation. Ensembl IDs were converted to gene names by BiomaRt R package. The genes with fold change > 2 and corrected p < 0.05 were screened as differential genes, through differential genes expression analysis with DESeq2 software.

### Data Processing and Analysis

Statistical analysis of data was perfomed by GraphPad Prism 7. The data shown in the graphs were mean ± variance or mean ± standard deviation. Please refer to reference signs of each figure for experiments for specific details. * represents p <0.05; * * represents p <0.01; * * * represents p <0.001; ns means no significant difference. Grayscale values of Western blot images were analyzed using ImageJ software to indicate protein abundance.

### Brief Description of the Drawings

The following reference drawings are for exemplary description of the present disclosure only. It is emphasized that the details shown are merely exemplary and illustrative of preferred embodiments of the present disclosure, aiming to describe the principles and concepts of the present disclosure. Those skilled in the art would readily understand how to practice the invention in its various forms with reference to the description of the drawings.

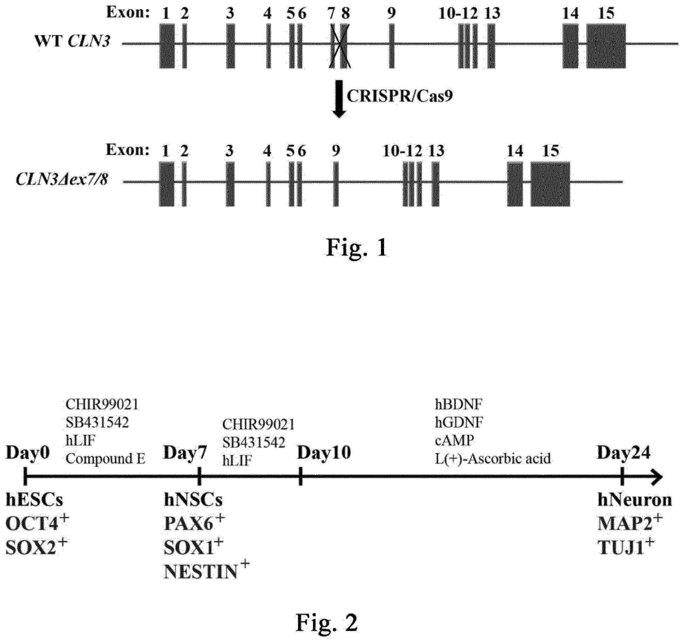

FIG. 1: JNCL patient mutations were introduced to human embryonic stem cells by CRISPR/Cas9 technology.
FIG. 2: Flowchart of differentiation of hESC cell lines into neural lineage.

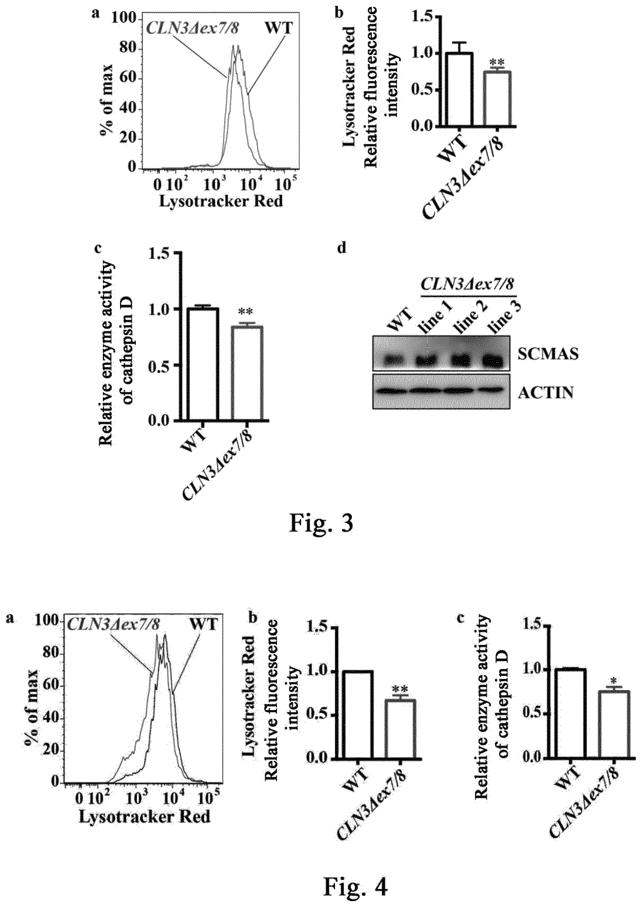

FIG. 3: Lysosomal functional impairment existed in *CLN3*^{*Δex7*/*8*} neural stem cells. Wherein (a) Lysotracker Red staining was performed in neural stem cells and intracellular red fluorescence intensity distribution was measured using flow cytometry; (b) Mean fluorescence intensity of Lysotracker staining in WT and *CLN3*^{*Δex7*/*8*} neural stem cells (n=3); (c) Comparison of cathepsin D activity in lysosomes of WT and *CLN3*^{*Δex7*/*8*} neural stem cells (n=4); (d) Western blot tests, for measuring subunit c of mitochondrial ATP synthase (SCMAS) abnormally accumulated in WT and *CLN3*^{*Δex7*/*8*} neural stem cells.
FIG. 4: Lysosomal functional impairment existed in *CLN3*^{*Δex7*/*8*} neurons. (a) Lysotracker Red staining was performed in neural stem cells and intracellular red fluorescence intensity distribution was measured using flow cytometry; (b) Mean fluorescence intensity of Lysotracker staining in WT and *CLN3*^{*Δex7*/*8*} neurons (n=3); (c) Comparison of cathepsin D activity in lysosomes of WT and *CLN3*^{*Δex7*/*8*} neurons (n=4).

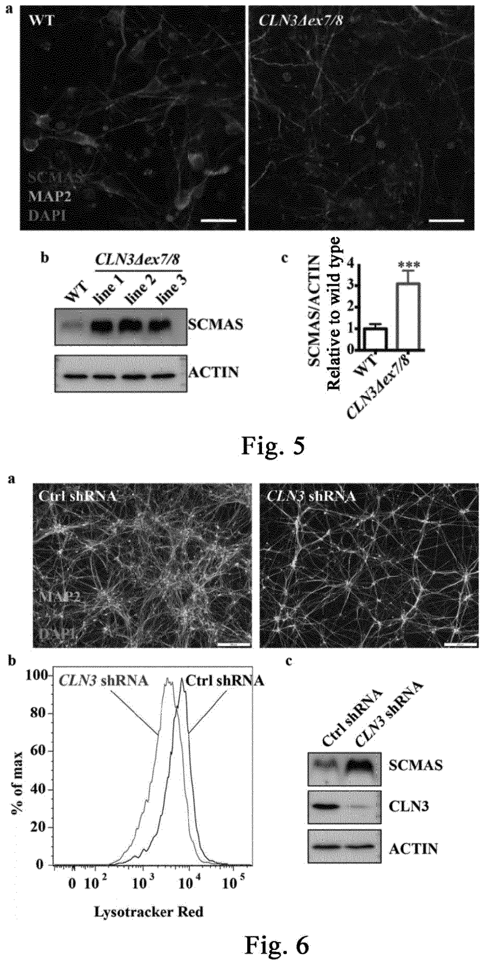

FIG. 5: An abnormal accumulation of SCMAS protein existed in *CLN3*^{*Δex7*/*8*} neurons. (a) Immunofluorescence staining revealed the presence of an abnormal accumulation of SCMAS protein within *CLN3*^{*Δex7*/*8*} neurons. The scale bar was 20 µm. (b) Western blot tests, for detecting the amount of SCMAS protein in WT and *CLN3*^{*Δex7*/*8*} neurons. (c) Quantification of the gray value of SCMAS bands in Western blot experiments (n=4).
FIG. 6: Lysosomal functional deficiencies existed in CLN3 knockdown neurons. (a) An equal number of hNSCs infected with control shRNA and CLN3 shRNA were differentiated into neurons. After 17 days of differentiation, immunofluorescence staining was used to detect the expression of neuronal lineage proteins MAP2 and TUJ1. The scale bar was 200 µm. (b) CLN3 knockdown neurons were stained with Lysotracker dye, and intracellular Lysotracker fluorescence intensity was measured by flow cytometry. (c) Western blot experiments, for measuring the protein amounts of CLN3 and SCMAS in neurons infected with control shRNA and CLN3 shRNA.

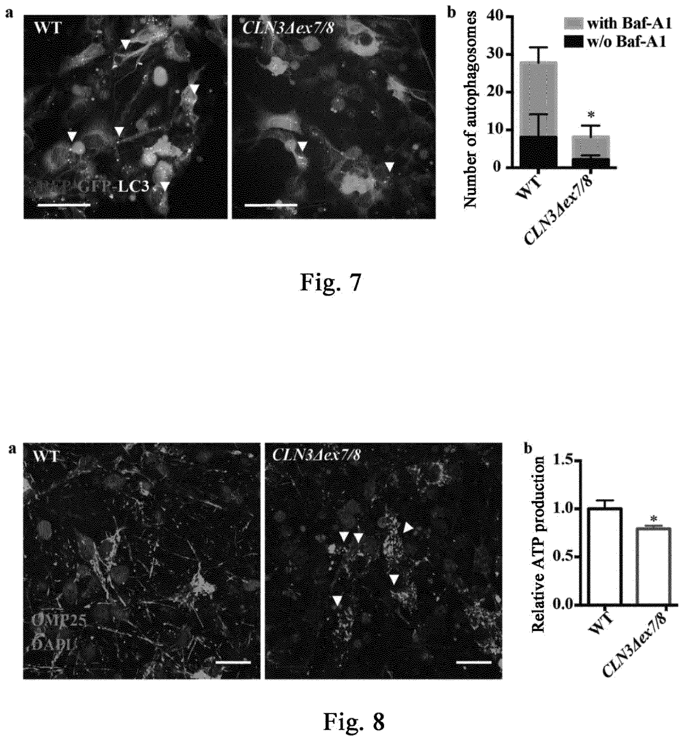

FIG. 7: Autophagic flux deficiencies existed in *CLN3*^{*Δex7*/*8*} neural stem cells. (a) Tandem LC3 lentivirus infected neural stem cells and labeled autophagosomes within the cells, followed by imaging through high-content laser confocal microscopy. White arrows pointed to single-positive autophagosomes with red fluorescence. The scale bar was 50 µm. (d) After treatment of neural stem cells with autophagy inhibitor Baf-A1 for 12 hours, the number of autophagosomes with red fluorescence in the cells was counted before and after Baf-A1 treatment. The figure showed the statistical results of 64 views by Opera Phenix data analysis workstation.
FIG. 8: Mitochondrial impairment existed in *CLN3*^{*Δex7*/*8*} neurons. (a) Green fluorescent fused OMP25 labeled mitochondrial morphology in human neurons. White arrows indicated impaired punctate mitochondria. The scale bar was 20 µm. (b) Cell Titer Glo detectrf ATP content in equal numbers of wild-type and *CLN3*^{*Δex7*/*8*} neurons (n=4).

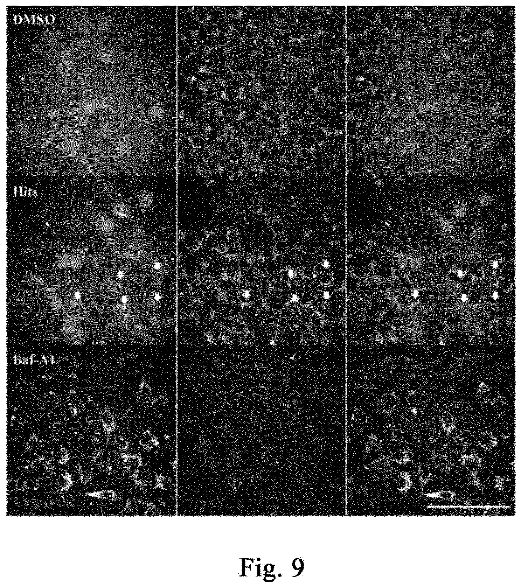

FIG. 9: Screening for small molecule compounds that enhance lysosomal acidity. Hits were representative images of small molecule compounds that enhanced the colouration of Lysotracker Red lysosomes. Arrows indicated autophagosomes colocalized with lysosomes after compound treatment. The scale bar was 100 µm.

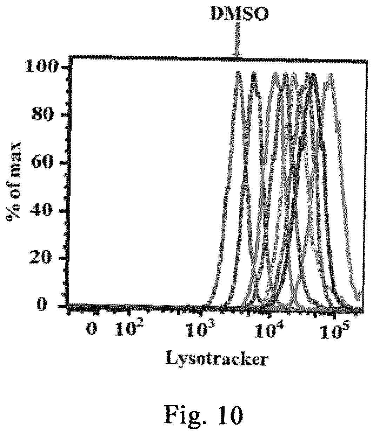

Fig. 10: Small molecule compounds enhanced lysosomal acidity.

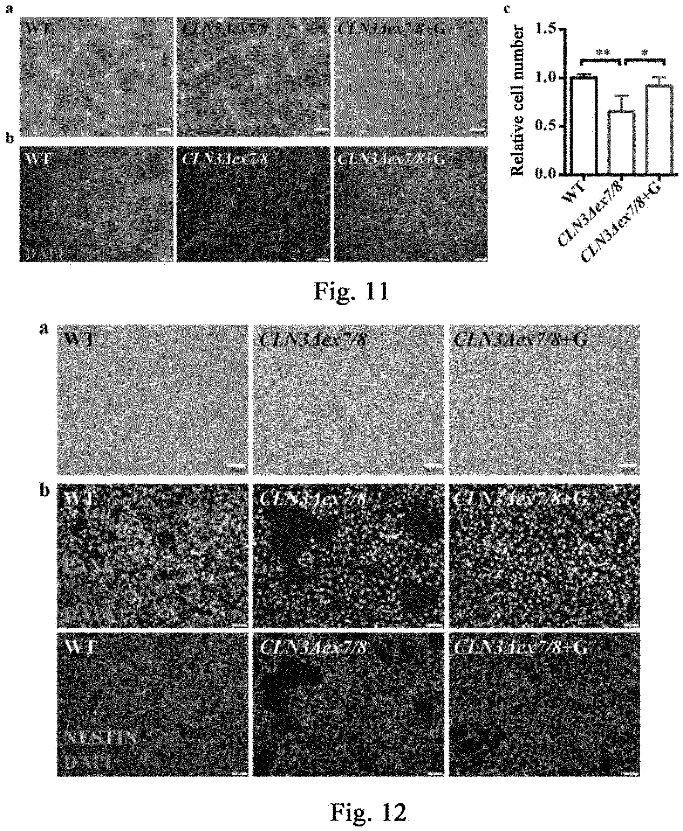

Fig. 11: Compound G promoted differentiation of *CLN3*^{*Δex7*/*8*} neurons. Equal numbers of neural stem cells were counted and took for initiating induced differentiation of neurons. (a) Bright field image after 17 days of differentiation. The scale bar was 200 µm. (b) Neurons differentiated for 17 Days were fixed followed by immunofluorescence staining of neuronal lineage markers MAP2 and TUJ1. The scale bar was 100 µm. (c) Relative cell number of differentiated neurons (n=4)
Fig. 12: Compound G did not affect the cell fate of *CLN3*^{*Δex7*/*8*} hNSC. (a) Bright field image of *CLN3*^{*Δex7*/*8*} hNSC treated with compound G for 48 hours. The scale bar was 200 µm. (b) hNSCs were fixed, followed by immunofluorescence staining of neural stem cell lineage proteins PAX6, SOX1 and NESTIN. The scale bar was 100 µm.

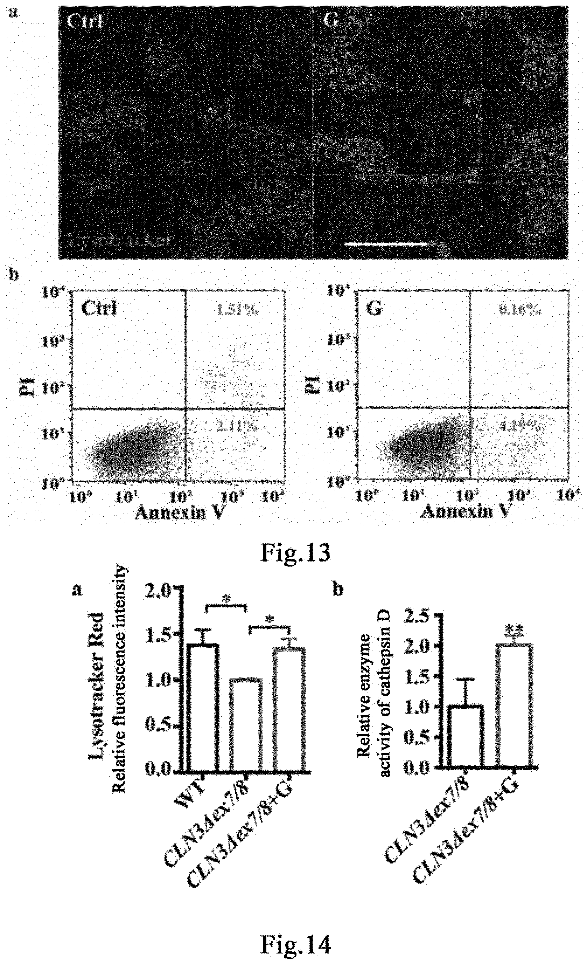

Fig. 13: Compound G increaseed lysosomal acidity in *CLN3*^{*Δex7*/*8*} hNSC. (a) After treatment with Compound B for 48h, *CLN3*^{*Δex7*/*8*} hNSC were stained with Lysotracker dye and imaged with a laser confocal high-content imaging microscope. The figure showed a mosaic of 9 adjacent fields. The scale bar was 200 µm. (b) After treatment with Compound B for 48h, *CLN3*^{*Δex7*/*8*} hNSC were stained with AnnexinV/PI apoptosis dye, followed by analysis through flow cytometry.
Fig. 14: Compound G alleviated lysosomal deficiencies in *CLN3*^{*Δex7*/*8*} human neurons. (a) Lysotracker staining was performed in neurons and mean fluorescence intensity of cells was measured by flow cytometry (n=3). (b) A substrate for cathepsin D was added to WT and CLN3Aex7/8 neuronal lysates, and the intensity of red fluorescence emitted after cleavage of the substrate by cathepsin D was measured with a microplate reader (n=4).

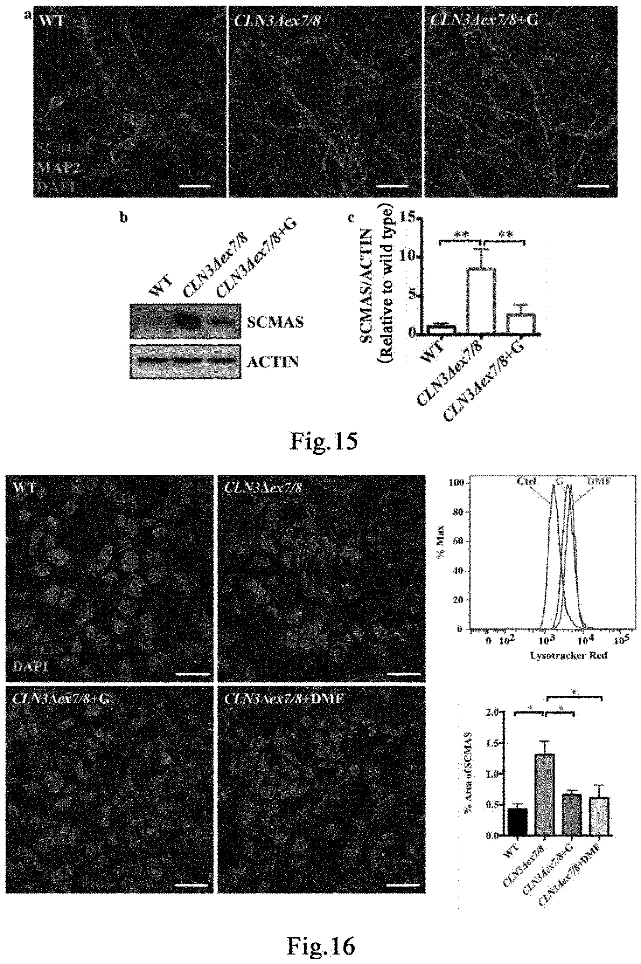

Fig. 15: Compound G reduced SCMAS protein storage in *CLN3*^{*Δex7*/*8*} neurons. (a) The abnormal accumulation of SCMAS protein in *CLN3*^{*Δex7*/*8*} neurons after treatment with Compound G was detected by immunofluorescence staining. The scale bar was 20 µm. (b) The effect of Compound G on the amount of SCMAS protein was measured by Western blot. (c) Grayscale values of SCMAS bands in Western blot were quantified (n=4).
Fig. 16: DMF reduced SCMAS protein storage in *CLN3*^{*Δex7*/*8*} neurons.

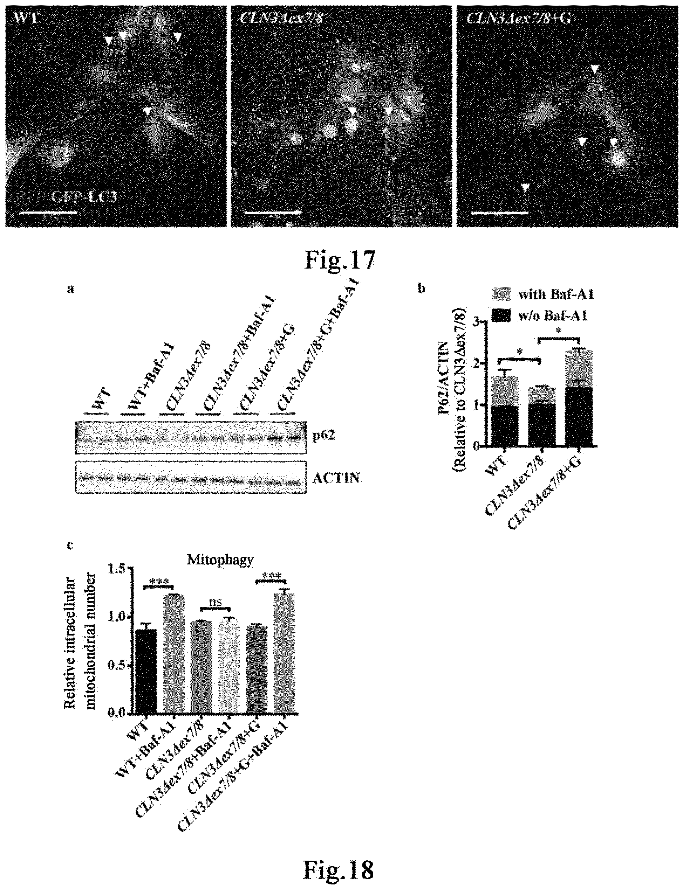

Fig. 17: Compound G enhanced autophagy flow in *CLN3*^{*Δex7*/*8*} human neural stem cells. White arrows pointed to red-fluorescent single-positive autophagosomes. The scale bar was 50 µm.
Fig. 18: Compound G enhanced autophagy flow in *CLN3*^{*Δex7*/*8*} human neurons. (a) Representative p62 protein blot image; (b) statistical graph of p62 protein levels (n=3). (c) Levels of intracellular mitophagy flow (n=6).

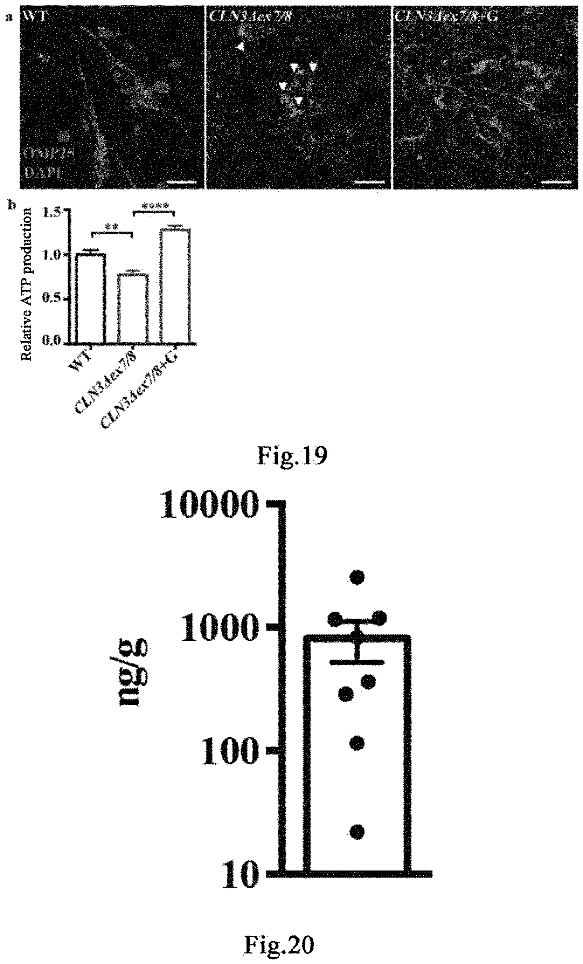

Fig. 19: Compound G protected mitochondrial homeostasis in *CLN3*^{*Δex7*/*8*} neurons. (a) White arrows pointed to impaired punctate mitochondria. The scale bar was 20 µm. (b) Equal numbers of neurons were counted and took, cell Titer Glo reagent was added, and intracellular ATP levels were measured with a microplate reader (n=3).
Fig. 20: Concentration of Compound G in mouse brain tissues (n=8).

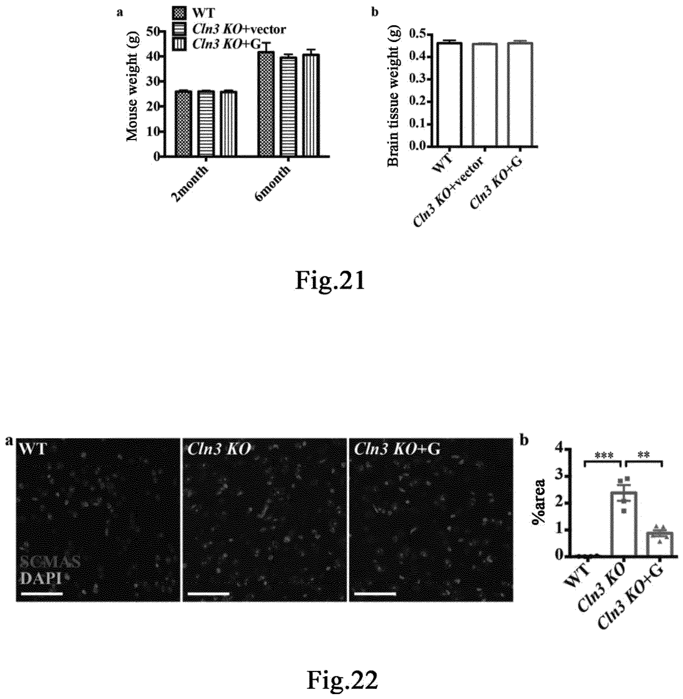

Fig. 21: The effect of Compound G on body weight and brain tissue quality in *Cln3 KO* mice. (a) The body weight of mice was recorded when they were 2 months and 6 months old (n=5). (b) The mice were euthanized when they were 6 months old, and the brain tissue was removed and weighed (n=5).
Fig. 22: Compound G reduced SCMAS protein accumulation in *Cln3 KO* mouse brain. (a) Wild-type and *Cln3 KO* mice at 2 months of age were euthanized, and the brain tissue thereof was removed for frozen section and immunohistochemical staining. The scale bar was 50 µm. (b) SCMAS fluorescence area was counted (WT: n=4; *cln3 KO*: n=4; *cln3 KO*+G: n=6).

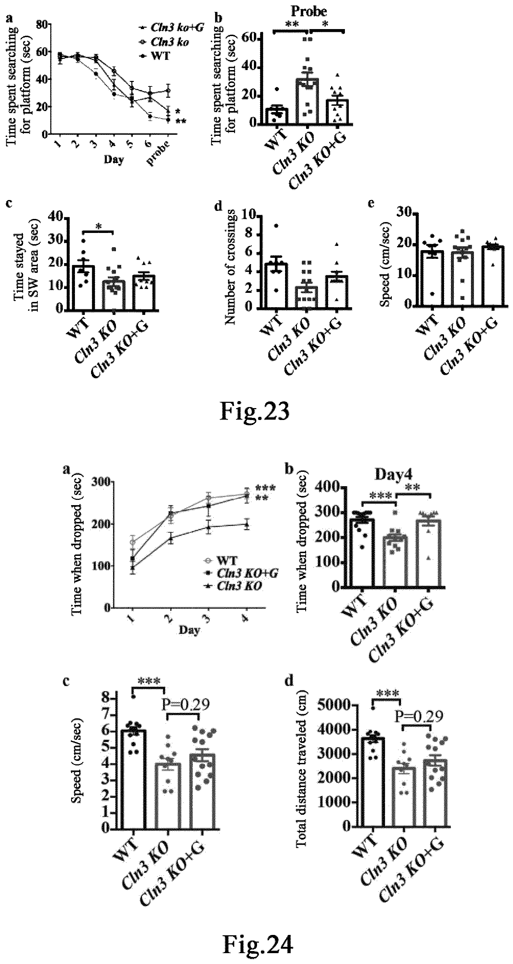

Fig. 23: The ability of learning and memory in *Cln3 KO* mice was measured by Morris water maze test. (a) 5-month-old mice were trained in a water maze for 6 days, and the time required to find the platform was recorded every day. On Day 7, the underwater platform was removed before tests. (b) The time required for the mice to first reach the position of the original platform when tested on Day 7. (c) The time the mouse stayed in the SW quadrant of the original platform when tested on Day 7. (d) The number of times the mice crossed the original platform location when tested on Day 7. (e) The mice's speed of movement in the water maze when tested on Day 7 (WT: n=8; *Cln3 KO*: n=13; *Cln3 KO*+G: n=10).
Fig. 24: The motor abilities of mice were tested by rotarod test and open field test. (a) The mice were trained for 3 days, the training duration was 300 seconds, and the time when the mice fell from the roller for the first time was recorded. (b) On day 4, the mice were tested and the time when the mice first fell from the roller was recorded. (c, d) The mice were gently placed into the open field and allowed to move freely for 10 minutes, and the length and movement speed of the mice within 10 minutes were recorded and analysed (WT: n=10; *Cln3* KO:n=10; *Cln3* KO+G: n=12).

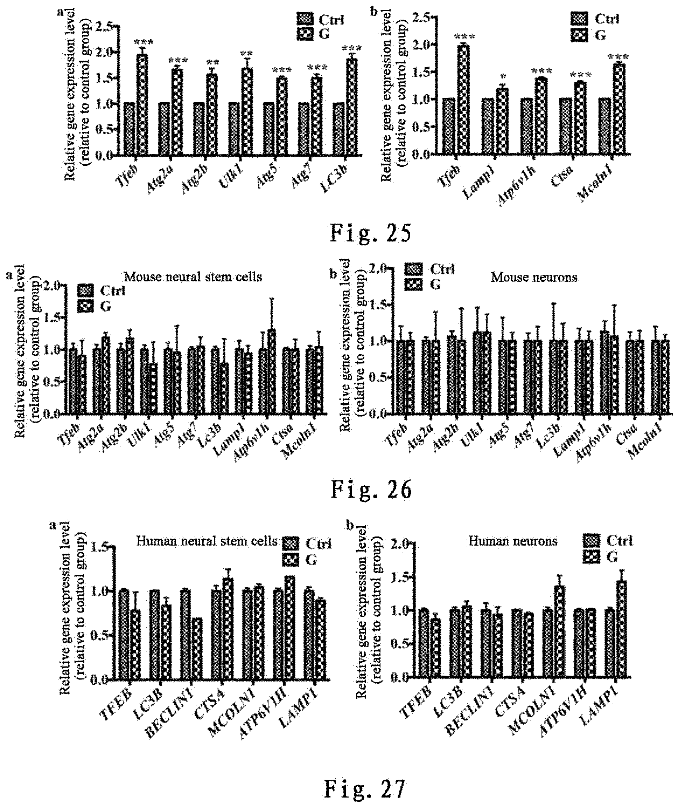

Fig. 25: Compound G increased transcription of autophagy and lysosomal genes in mouse TTF cells. The transcription levels of autophagy- and lysosome-related genes were detected using qRT-PCR (n=3).
Fig. 26: Compound G did not affect autophagy and lysosomal gene transcription in mouse neural stem cells and neurons. After treating primary (a) neural stem cells and (b) neurons of *Cln3 KO* mice for 6 hours, the mRNA was extracted from the cells, reverse-transcribed into cDNA, and transcription levels of autophagy- and lysosome-related genes were detected using qRT-PCR (n=3).
Fig. 27: Compound G did not affect autophagy and lysosomal gene transcription in human neural stem cells and neurons. After treating (a) human neural stem cells and (b) neurons with Compound G for 6 hours, the mRNA was extracted from the cells, reverse-transcribed into cDNA, and transcription levels of autophagy- and lysosome-related genes were detected using qRT-PCR (n=3).

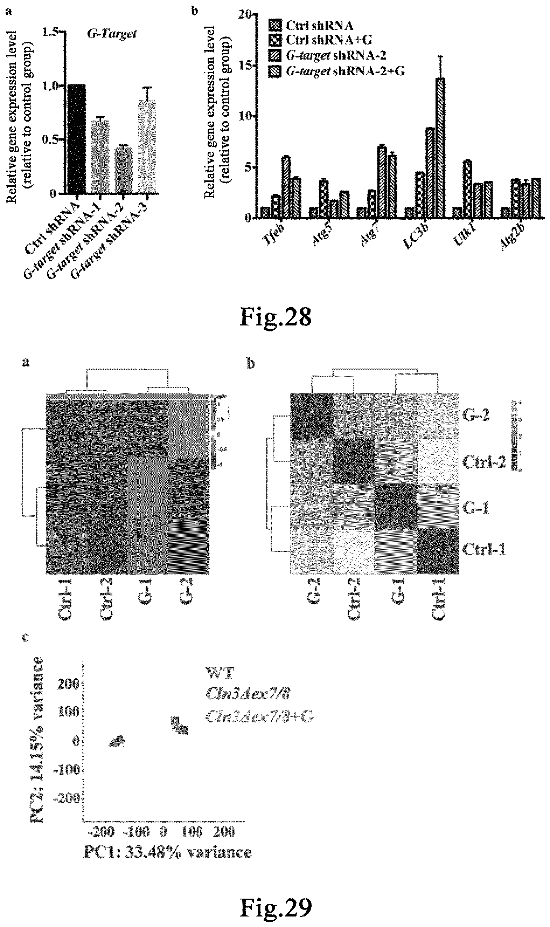

Fig. 28: Compound G increases transcription of autophagy genes in a FXR-dependent manner. (a) The mRNA was extracted from mouse TTF cells, and the expression level of *FXR* was detected by qRT-PCR (n=3). (b) Mouse TTF cells expressing control shRNA or *FXR* shRNA-2 were treated with Compound G for 6 hours, and the expression levels of Tfeb and autophagy-related genes in the cells were detected by qRT-PCR (n=3).
Fig. 29: The effect of Compound G on the expression profile of *CLN3*^{*Δex7*/*8*} human neural stem cells. *CLN3*^{*Δex7*/*8*} human neural stem cells were treated with Compound G for 6 hours, and cellular mRNA was extracted for library construction and RNA-seq tests (n=2). (a) A cluster heat map of differentially expressed genes. (b) A distance figure. (c) A PCA figure.

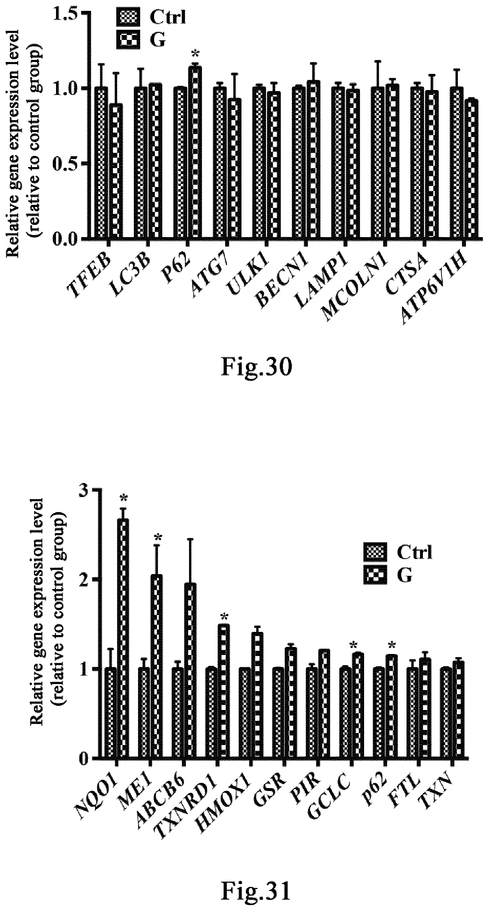

Fig. 30: RNA-seq showed that Compound G did not affect autophagy and lysosomal gene expression in neural stem cells. *CLN3*^{*Δex7*/*8*} human neural stem cells were treated with Compound G for 6 hours and RNA-seq was performed to analyze the expression level of lysosomal and autophagy-related genes in the sequencing data (n=2).
Fig. 31: RNA-seq data showed that Compound G activated expression of genes downstream of NRF2. *CLN3*^{*Δex7*/*8*} human neural stem cells were treated with Compound G for 6 hours and RNA-seq was performed to analyze the expression level of genes downstream of NRF2 in the sequencing data (n=2).

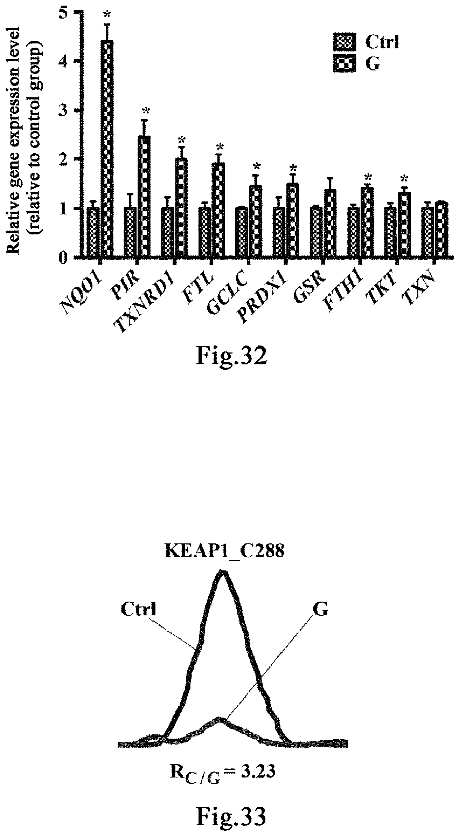

Fig. 32: Compound G activated expression of NRF2 downstream genes in *CLN3*^{*Δex7*/*8*} human neural stem cells. *CLN3*^{*Δex7*/*8*} human neural stem cells were treated with Compound G for 6 hours and subjected to qRT-PCR to detect the expression of NRF2 downstream genes (n=3).
Fig. 33: The quantitative thiol reactivity analysis in *CLN3*^{*Δex7*/*8*} human neural stem cells.

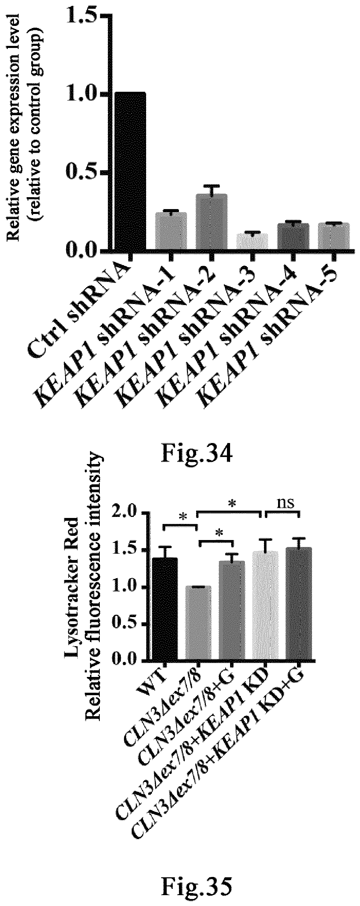

Fig. 34: The *KEAP1* knockdown was performed in *CLN3*^{*Δex7*/*8*} human neural stem cells.
Fig. 35: The effect of *KEAP1* knockdown on *CLN3*^{*Δex7*/*8*} human neuronal lysosomal acidity.

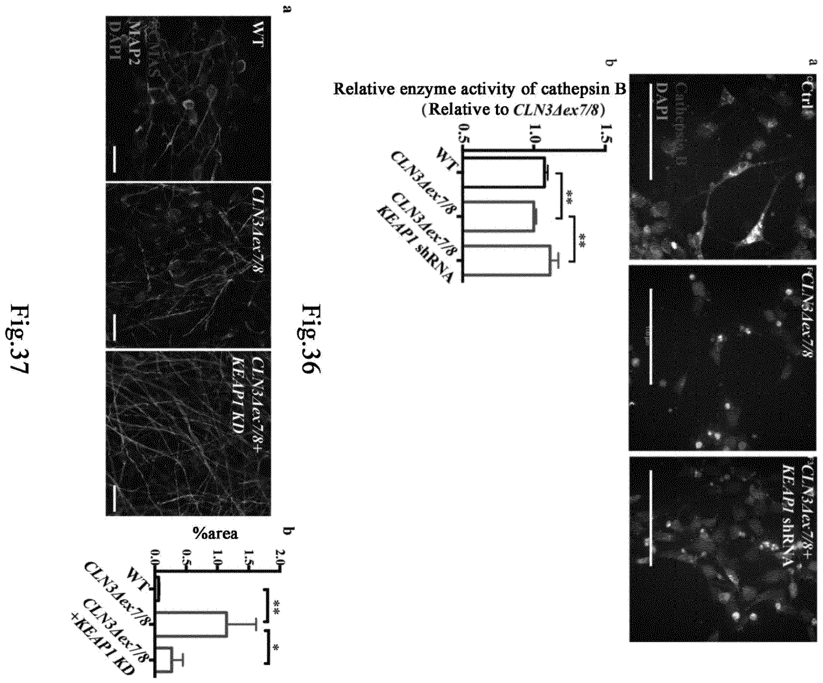

Fig. 36: The effect of knockdown of *KEAP1* on cathepsin activity in *CLN3*^{*Δex7*/*8*} cells. (a) Cell imaging was performed using laser confocal high-content imaging microscopy to observe the intensity of intracellular red fluorescence. The scale bar was 100 µm. (b) Intracellular red fluorescence intensity was counted using the Opera Phenix data analysis workstation, and the figure showed the statistical results of 528 views.
Fig. 37: The knockdown of *KEAP1* reduced SCMAS protein storage in *CLN3*^{*Δex7*/*8*} neurons. (a) Immunofluorescence staining showed the effect of *KEAP1* knockdown on the abnormal accumulation of SCMAS protein in *CLN3*^{*Δex7*/*8*} neurons. The scale bar was 20 µm. (b) SCMAS fluorescence areas were counted (n=4).

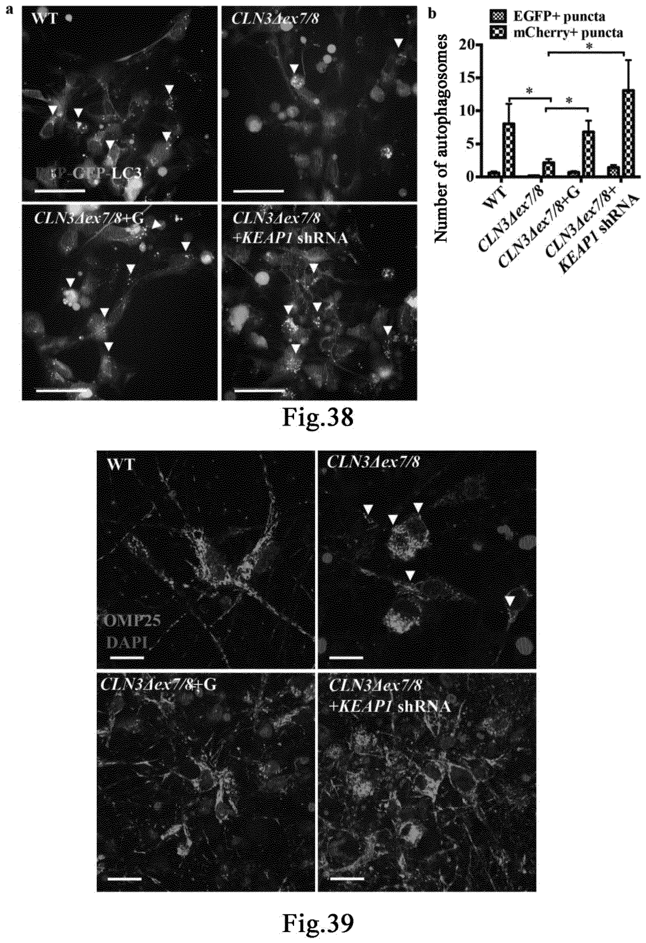

Fig. 38: The effect of knockdown of *KEAP1* on autophagy flow in *CLN3*^{*Δex7*/*8*} human neural stem cells. (a) White arrows pointed to red fluorescent single positive autophagosomes. The scale bar was 50 µm. (b) The figure showed the statistical results of 64 views by Opera Phenix data analysis workstation. Number of red fluorescent single positive autophagosomes = (number of mCherry positive autophagosomes - number of EGFP positive autophagosomes).
Fig. 39: Knockdown of *KEAP1* reduced mitochondrial imparation in *CLN3*^{*Δex7*/*8*} human neurons. White arrows pointed to impaired punctate mitochondria. The scale bar was 20 µm.

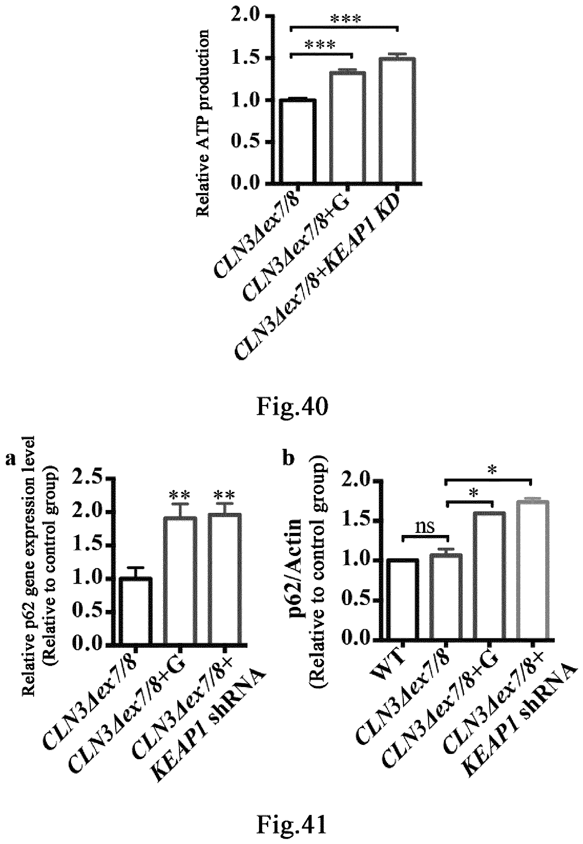

Fig. 40: Knockdown of *KEAP1* increased ATP content in *CLN3*^{*Δex7*/*8*} human neurons. An equal number of neurons were counted and token using a hemocytometer, Cell Titer Glo reagent was added, and intracellular ATP levels were measured using a microplate reader (n=6).
Fig. 41: Compound G increased intracellular *p62* protein levels, (a) 24 hours after treatment of human neurons with Compound G or after *KEAP1* knockdown, transcriptional levels of the *p62* gene were detected by qRT-PCR (n=3). (b) 24 hours after treatment of human neurons with Compound G or after *KEAP1* knockdown, intracellular *p62* protein levels were detected by Western blotting and band intensity statistics (n = 3) were performed.

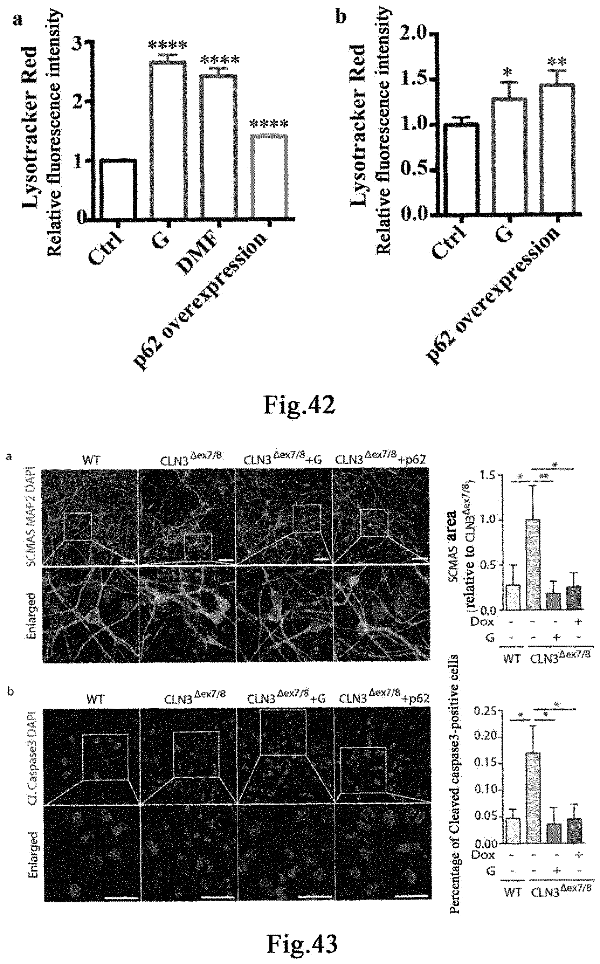

Fig. 42: Overexpression of p62 protein increaseed lysosomal acidity. (a) GFP-p62 protein and control GFP protein were overexpressed in NRK cells and after 48 hours, stained with Lysotracker stain and the intensity of red fluorescence within green-fluorescence-positive cells was detected by flow cytometry (n=3). (b) GFP-p62 protein and control GFP protein were overexpressed in human neural stem cells and after 48 hours, stained with Lysotracker stain and the intensity of red fluorescence within green-fluorescence-positive cells was detected by flow cytometry (n=3).
Fig. 43: Overexpression of p62 protein reduced SCMAS protein accumulation and cell death in *CLN3*^{*Δex7*/*8*} neurons. DOX was used to overexpress p62 protein in *CLN3*^{*Δex7*/*8*} human neural stem cells and differentiate them into human neurons. (a) Immunofluorescence staining showed the effect of p62 overexpression on abnormal accumulation of SCMAS protein in *CLN3*^{*Δex7*/*8*} neurons. The scale bar was 20 µm. (b) Immunofluorescence staining showed the effect of p62 overexpression on apoptosis of *CLN3*^{*Δex7*/*8*} neurons. The scale bar was 10 µm.

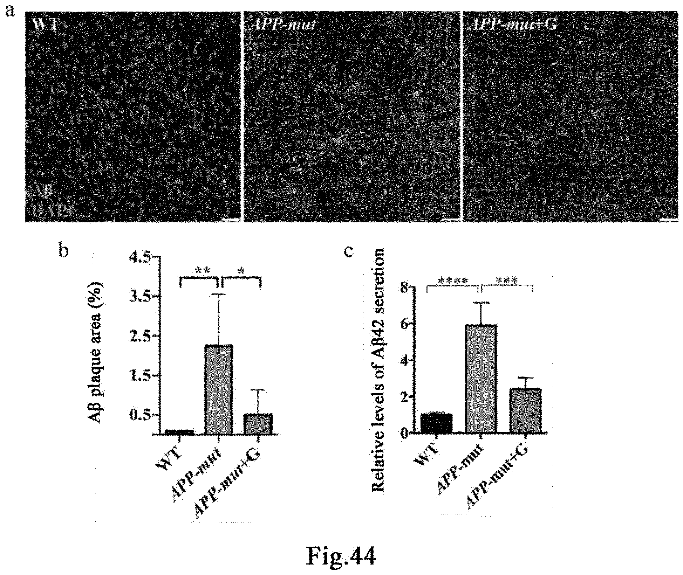

Fig. 44: Compound G reduced Aβ protein storage and secretion levels of toxic Aβ42 in *APP* mutant neurons. (a) Immunofluorescence staining, for detecting Compound G-treated *APP* mutant neurons for abnormal accumulation of Aβ protein and (b) statistics of Aβ plaque area, and the scale bar was 50 µm. (c) ELISA tests, for measuring the effect of Compound G on the secretion level of toxic Aβ42 of *APP* mutant neurons.

### EXAMPLES

The present disclosure will be further described in detail below with reference to the following examples, which are only to illustrate the present disclosure and should not be considered as limiting the scope of the present disclosure. Modifications or improvements made on the basis of not deviating from the present disclosure are all included herein within the scope of protection of the present disclosure. Unless otherwise stated, the experimental methods used in the following examples are all conventional methods; the reagents and materials used are all commercially available.

### Example 1: Construction of CLN3^{Δex7/8} cell line

To construct an *in vitro* JNCL human neuronal cell model, homozygous deletion of exons 7 and 8 of *CLN3* (*CLN3*^{*Δex7*/*8*}), the most common mutation in JNCL patients, was introduced into H9 human embryonic stem cells using CRISPR/Cas9 gene editing technology with reference to the disclosed method (Ran et al., 2013) (Figure 1). The primers used in the process of construction are shown in Table 7, and the other steps are the same as in Ran et al.

**Table 7: Primers for CLN3^{Δex7/8} cell line construction**

| Name | Forward primers | Reverse primers |
|---|---|---|
| sgRNA-1 | CACCGAACCTAAGTGACTAAAATCG | AAACCGATTTTAGTCACTTAGGTTC |
| sgRNA-2 | CACCGCAGTAGGCGATGGGAGACT | AAACAGTCTCCCATCGCCTACTGC |
| up_arm | TGCAACTCTGTCTCTACGGC | AGCAGGCTTCTAAGGGTGAC |
| down_arm | TGGGAGCTAGAGCGACCATT | CGGTATTGCTGAGCGTGACT |
| CLN3_WT | AGCTGATACTGAGGAGGCCA | GGGATACCCAGCATGGACAG |
| Puro | ATGACCGAGTACAAGCCCAC | TCAGGCACCGGGCTTGCG |
| CLN3_mut | AGCTCTGCGGTCTCACTCTA | CTGGCTCATACCTAGCGAGTC |

### Example 2: Construction of human neuronal cell model of JNCL disease

This study confirms that the patient's nervous system, especially the homeostasis and survival of neurons, is most affected by mutations in the *CLN3* gene. Directed differentiation of *CLN3*^{*Δex7*/*8*} hESC into the neural lineage was induced *in vitro* to construct the human neuronal cell model of JNCL disease. This model recapitulates several JNCL-associated phenotypes, such as lysosomal and autophagy functional defects and mitochondrial homeostasis dysregulation.

The method for efficiently differentiating hESC into neural stem cells and neurons was performed as shown in Figure 2. Firstly, CHIR99021, SB431542, hLIF, and Compound E were used to induce hESC to differentiate into neural lineage. After 7 days of induction, both wild-type and *CLN3*^{*Δex7*/*8*} hESC were efficiently induced to become PAX6, SOX1, and NESTIN-positive human neural stem cells (hNSC). hNSC were then treated with CHIR99021, SB431542, and hLIF for 3 days, followed by changing the culture medium to contain hBDNF, hGDNF, cAMP, and vitamin C, and continuing for another 14 days of induction. MAP2 and TUJ1-positive human neurons were then obtained.

Immunofluorescence staining showed that, after 7 days of differentiation, both wild-type and *CLN3*^{*Δex7*/*8*} hESC were efficiently induced to PAX6, SOX1, and NESTIN-positive human neural stem cells. Lineage gene expression analysis further demonstrated that, compared with hESC, both wild-type and *CLN3*^{*Δex7*/*8*} neural stem cells highly expressed the neural stem cell linage genes *PAX6*, *SOX1*, and *NOGGIN*, and the expression level of *OCT4*, an embryonic stem cell pluripotency gene, was significantly decreased.

### Example 3: In vitro recapitulation of disease-associated phenotypes in CLN3^{Δex7/8} human neural stem cells and neurons

The effect of homozygous deletion of exons 7 and 8 of *CLN3* gene on the homeostasis of human neural stem cells and neuronal cells was determined in terms of lysosomal acidity, protease activity, intracellular protein storage, autophagic flux and mitochondrial dysfunction, etc. in the cells obtained by differentiation.

### Lysosomal defects in CLN3^{Δex7/8} human neural stem cells

The cells were stained with Lysotracker, a fluorescent dye that is enriched in acidic lysosomes and emits red fluorescence. The fluorescence intensity of the stained cells was then analyzed using flow cytometry.

The results showed (Figure 3) that, compared to wild-type human neural stem cells, the average fluorescence intensity of Lysotracker in *CLN3*^{*Δex7*/*8*} cells was significantly decreased, i.e., the overall decrease in cellular lysosomal acidity, suggesting that the mutation of the CLN3 protein leads to a dysregulation of lysosomal pH regulation in neural stem cells. The protein quantities of SCMAS in neural stem cells were examined by western blot. The results showed that there was abnormal storage of SCMAS in *CLN3*^{*Δex7*/*8*} neural stem cells, indicating that it could not be successfully degraded within the lysosome.

In conclusion, the *CLN3* gene mutation in neural stem cells obtained by differentiation can result in JNCL-associated lysosomal defects such as lysosomal acidity dysregulation, protease activity reduction, and abnormal protein storage.

### Lysosomal defects in CLN3^{Δex7/8} human neurons

Lysosomal and autophagy functions were further examined in *CLN3*^{*Δex7*/*8*} neurons. After differentiating wild-type and *CLN3*^{*Δex7*/*8*} human neural stem cells into neurons, the neurons were first stained with Lysotracker Red dye, and the fluorescence intensity of wild-type and *CLN3*^{*Δex7*/*8*} neurons was then analyzed by flow cytometry, respectively. Consistent with the results observed in neural stem cells, compared with wild-type neurons, *CLN3*^{*Δex7*/*8*} neurons had a phenotype of decreased lysosomal acidity (Figure 4 (a, b)); and the enzymatic activity of cathepsins D in the lysosomes of *CLN3*^{*Δex7*/*8*} neurons was significantly decreased (Figure 4 (c)). The cellular immunofluorescence and western blot together demonstrated that there was a significant amount of abnormal storage of SCMAS protein in *CLN3*^{*Δex7*/*8*} human neurons (Figure 5).

The above results indicated that, similar to human neural stem cells, *CLN3*^{*Δex7*/*8*} human neurons also exhibited the phenotypes of lysosomal function defects, such as lysosomal acidity dysregulation, cathepsins activity reduction, and abnormal storage of undegraded proteins.

To further determine that the lysosomal defects observed in *CLN3*^{*Δex7*/*8*} human neurons described above are due to the loss-of-function of the CLN3 protein, *CLN3* knockdown was performed in wild-type neural stem cells using short hairpin RNA (shRNA) targeting the *CLN3* transcript, and wild-type neural stem cells infected with target-free shRNA (Ctrl shRNA) were used as a control, which were both differentiated into human neurons later. The changes of CLN3 protein level in neurons expressing *CLN3* shRNA were analyzed by western blot. The results showed that the use of *CLN3* shRNA significantly reduced the CLN3 protein expression level in neurons compared to the control shRNA (Figure 6 (c)).

In addition, equal amounts of neural stem cells expressing *CLN3* shRNA and control shRNA were seeded into new cell culture plates and induced to differentiate toward human neurons by the method described above. After 17 days of differentiation, immunofluorescence staining showed that *CLN3* knockdown neural stem cells could be successfully differentiated into MAP2 and TUJ1 positive neurons (Figure 6 (a)). Consistent with the results observed in *CLN3*^{*Δex7*/*8*} cells, starting from the same amount of cells, the cell density of neurons obtained from the differentiation of *CLN3* knockdown neural stem cells was significantly decreased (Figure 6 (a)).

The effect of *CLN3* knockdown on lysosomal acidity in human neurons was examined using Lysotracker dye. The results showed that Lysotracker staining intensity was weakened in neurons after *CLN3* knockdown, indicating a decrease in their lysosomal acidity (Figure 6 (b)). It was observed using western blot that *CLN3* knockdown can lead to abnormal storage of SCMAS protein in neurons (Figure 6 (c)).

Together, these results suggest that functional deficiency of CLN3 protein in human neurons can cause lysosomal dysfunction phenotypes such as lysosomal acidity dysregulation and abnormal protein storage, which affect cellular homeostasis.

### Autophagy defects in CLN3^{Δex7/8} human neurons

After lysosomal function defects were detected in *CLN3*^{*Δex7*/*8*} neural stem cells and neurons, autophagic flux in the cells was further examined. Equal amounts of lentivirus packaged with tandem LC3 plasmid were used to infect wild-type and *CLN3*^{*Δex7*/*8*} cells to label intracellular autophagosomes. Compared with wild-type neural stem cells, the number of red fluorescent single-positive autophagosomes was decreased in *CLN3*^{*Δex7*/*8*} human neural stem cells (indicated by arrows in Figure 7 (a)), suggesting that there may be a blockage of autophagic flux in the cells.

The number of autophagosomes in the cells was counted before and after treatment with the autophagy blocker bafilomycin A1 (Baf-A1). The results showed that the changes of the number of autophagosomes in wild-type neural stem cells (number of autophagosomes after treatment with Baf-A1 - number of autophagosomes before treatment with Baf-A1) was significantly higher than that in *CLN3*^{*Δex7*/*8*} neural stem cells (Figure 7 (b)), confirming that autophagic flux in *CLN3*^{*Δex7*/*8*} human neural stem cells was defective.

Studies have shown that healthy mitochondria have a short stick-like morphology, with large potential differences across the mitochondrial membrane, which allows efficient generation of ATP that maintains normal cellular function. Damaged mitochondria undergo the process of mitochondrial fission, in which the mitochondria split into a punctate morphology, encapsulated by autophagosome, and transported into lysosomes for degradation. To detect the presence of abnormal mitochondrial morphology in *CLN3*^{*Δex7*/*8*} human neurons, a Lentivirus plasmid with green fluorescent protein labeled OMP25 was constructed and used to infect wild-type and *CLN3*^{*Δex7*/*8*} human neurons

The results showed that the mitochondria in wild-type human neurons were mostly healthy short stick-shaped. In contrast, there were a large number of punctate mitochondria in *CLN3*^{*Δex7*/*8*} human neurons (Figure 8 (a)), suggesting severe mitochondrial damage in *CLN3*^{*Δex7*/*8*} human neurons. The ATP generation capacity in *CLN3*^{*Δex7*/*8*} human neurons was further examined using Cell Titer Glo reagent. Quantitative determination with the plate reader revealed that the level of ATP in *CLN3*^{*Δex7*/*8*} human neurons with impaired autophagic flux was significantly lower compared to an equal number of wild-type human neurons (Figure 8 (b)).

Together, the above data suggest that defects of lysosomal and autophagy functions are present in neural stem cells and neurons differentiated *in vitro* from *CLN3*^{*Δex7*/*8*} human embryonic stem cells and affect the homeostasis and function of mitochondria in the cells.

### Example 4: The compounds of the present disclosure are autophagy inducers capable of increasing lysosomal acidity

The inventors constructed a normal rat kidney (NRK) cell line that stably expresses tandem LC3. The tandem LC3 system is an LC3 reporter system that uses both red and green fluorescent labels. When the autophagosomes with the dual fluorescently labeled LC3 are located in the cytoplasm, the autophagosomes simultaneously show red fluorescence and green fluorescence (yellow). As the autophagosomes fuse with lysosomes to form an autolysosome, the acidic environment in lysosomes quenches the green fluorescence of tandem LC3, and thus autolysosome only present red fluorescence (Shunsuke Kimura et al., 2007). Thereby, this system is able to distinguish between autophagy inducers and lysosomal inhibitors.

The inventors obtained seven small molecule compounds which were able to increase the number of red fluorescent and green fluorescent double-positive autophagosomes in cells by two-fold and more simultaneously, and to keep the number of red fluorescent single-positive autophagosomes from decreasing, when tested with the tandem LC3 system described above, suggesting that all of them are autophagy inducers rather than lysosomal inhibitors. Moreover, after treating BFP-LC3 stably expressing NRK cells with these seven small molecule compounds for 18 hours, staining with Lysotracker dye was performed. It could be observed that, compared with control cells, these seven small molecule compounds could significantly increase intracellular Lysotracker staining (Figures 9, 10), indicating that they increased intracellular lysosomal acidity. As a control, Baf-A1 treatment resulted in a significant decrease in lysosomal acidity, with the acidophilic Lysotracker dye failing to locate properly in the lysosome and showing a dispersive state in the cytoplasm (Figure 9).

Said seven small molecule compounds are:

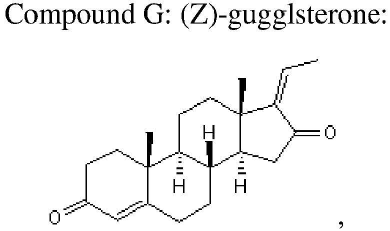

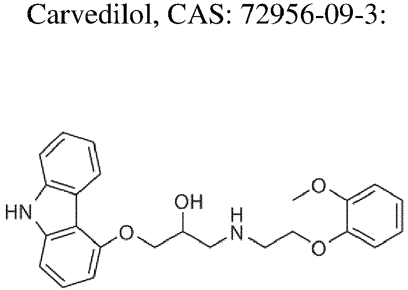

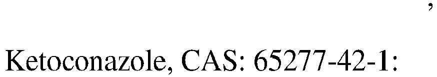

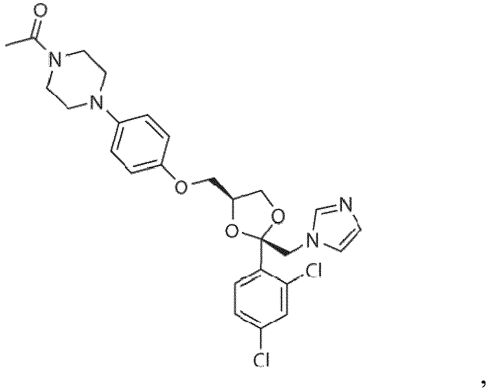

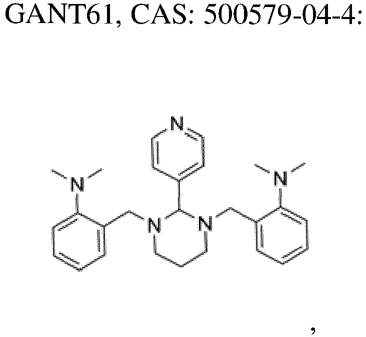

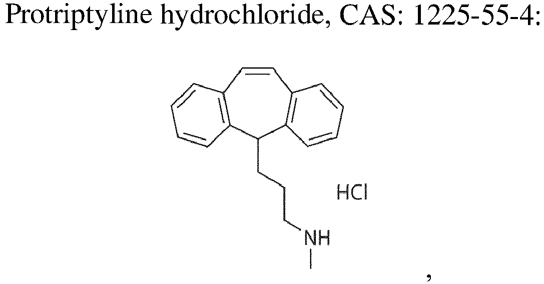

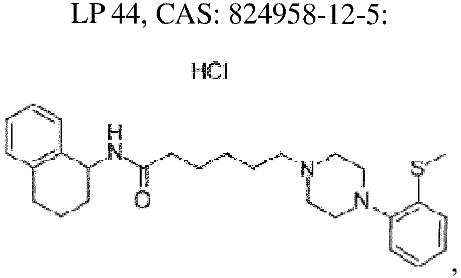

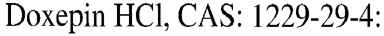

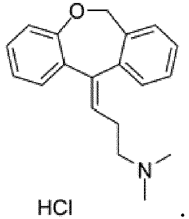

Based on the results of the above tests, those small molecule compounds that increase cellular autophagic flux are all potential compounds that can be used in the treatment of JNCL disease. Compound G was next verified as an example.

### Example 5: Compound G alleviates disease phenotypes in JNCL human neural cell model

The inventors tested the potential effects of the previously mentioned small molecule compounds on JNCL using compound G as an example.

### Compound G alleviates the differentiation defects in CLN3^{Δex7/8} neural stem cells

Starting from the same number of human neural stem cells induced to differentiate into human neurons, *CLN3*^{*Δex7*/*8*} neural stem cells eventually generated a lower number of neurons than wild-type cells. During the process of neurons differentiation, the seven small molecule compounds obtained above were each added to the culture medium and observed for their effects on the differentiation process of *CLN3*^{*Δex7*/*8*} neural stem cells into neurons.

After 17 days of differentiation, Compound G was found to be effective in alleviating the differentiation defects of *CLN3*^{*Δex7*/*8*} neural stem cells to neurons. Starting from the same number of cells, Compound G-treated *CLN3*^{*Δex7*/*8*} neural stem cells were able to generate more MAP2 and TUJ1-positive neurons, which were close to the density of neurons generated by differentiation from wild-type neural stem cells (Figure 11).

### Compound G: (Z)-gugglsterone, with the following structural formula:

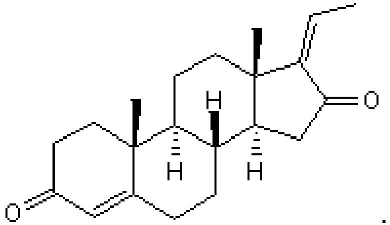

A further examination of the effects of Compound G on lysosomal function, autophagy function and cellular homeostasis in *CLN3*^{*Δex7*/*8*} neurons was performed.

After 48 hours of treatment of *CLN3*^{*Δex7*/*8*} neural stem cells with Compound G, immunofluorescence staining was performed for the neural stem cell lineage markers SOX1, PAX6 and NESTIN. The results showed that Compound G-treated *CLN3*^{*Δex7*/*8*} neural stem cells maintained the characteristics of SOX1, PAX6 and NESTIN positive (Figure 12), indicating that Compound G treatment would not change the cell fate of *CLN3*^{*Δex7*/*8*} neural stem cells.

Then, whether compound G causes cytotoxicity in neural stem cells was examined. Cell apoptosis was detected after compound G treatment with Annexin V and propidium iodide (PI) staining and analyzed with flow cytometry. Annexin V positive and PI negative represented early apoptotic cells, while Annexin V positive and PI positive represented late apoptotic cells. The results showed that the background proportions of early apoptotic cells and late apoptotic cells in *CLN3*^{*Δex7*/*8*} neural stem cells were not high (Figure 13 (b)), and the treatment of Compound G for 48 hours did not significantly change the proportions of apoptotic cells in *CLN3*^{*Δex7*/*8*} neural stem cells, which indicated that the treatment of Compound G did not exhibit significant cytotoxicity to *CLN3*^{*Δex7*/*8*} neural stem cells.

After staining with Lysotracker dye in *CLN3*^{*Δex7*/*8*} neural stem cells, it could be seen that Compound G could significantly improve the Lysotracker staining in neural stem cells (Figure 13 (a)), suggesting that Compound G could also significantly enhance the acidic environment of intracellular lysosomes in human neural stem cells.

### Compound G alleviates the lysosomal and autophagy defects in CLN3^{Δex7/8} neurons

Studies above have shown that *CLN3*^{*Δex7*/*8*} human neurons feature lysosomal acidity dysregulation. To investigate the effect of Compound G on lysosomal acidity in *CLN3*^{*Δex7*/*8*} neurons, Lysotracker dye was used for staining and flow cytometry was used for analyzing the fluorescence intensity of Lysotracker dye in cells. The results showed that the Lysotracker fluorescence intensity in *CLN3*^{*Δex7*/*8*} human neurons increased significantly after Compound G treatment (Figure 14 (a, b)), which was much closer to that in wild-type neurons, indicating that the treatment of Compound G could increase the lysosomal acidity of *CLN3*^{*Δex7*/*8*} neurons.

The activity of cathepsins D was detected in *CLN3*^{*Δex7*/*8*} neurons after treatment with compound G. The results showed that the fluorescence intensity was significantly increased in *CLN3*^{*Δex7*/*8*} neurons after treatment with compound G, which was much closer to that in wild-type neurons, indicating that the activity of cathepsins D was enhanced (Figure 14 (b)). Immunofluorescence staining and western blot showed that treatment with compound G resulted in a significant reduction of abnormal storage of SCMAS protein in *CLN3*^{*Δex7*/*8*} neurons (Figure 15).

It can be seen that the treatment of the screened compound G can effectively alleviate the lysosomal acidity dysregulation in *CLN3*^{*Δex7*/*8*} human neurons, increase the lysosomal protease activity, and reduce the abnormal intracellular protein storage, i.e., the compound G can alleviate the JNCL disease-associated phenotypes to a certain extent in *CLN3*^{*Δex7*/*8*} human neuronal models.

The inventors also tested several other potentially useful compounds and found that, similar to Compound G, they were also capable of alleviating the JNCL disease-associated phenotypes to some extent in the *CLN3*^{*Δex7*/*8*} human neuronal model. For example, the inventors tested dimethyl fumarate (DMF): using Lysotracker staining to characterize the lysosomal acidity in the cells and analyzing the average fluorescence intensity of the staining using flow cytometry. The inventors found that the average fluorescence intensity of the Lysotracker in the DMF-treated *CLN3*^{*Δex7*/*8*} human neurons was significantly increased, suggesting that DMF can increase the lysosomal acidity in *CLN3*^{*Δex7*/*8*} human neurons. Tests for the effect of DMF on SCMAS protein storage in *CLN3*^{*Δex7*/*8*} human neurons showed that DMF treatment significantly reduced SCMAS protein storage in *CLN3*^{*Δex7*/*8*} human neurons (Figure 16).

### Compound G increases the level of autophagy and mitochondrial autophagy in CLN3^{Δex7/8} neurons and maintains the function and homeostasis of mitochondria

Autophagosomes in neural stem cells were labeled using tandem LC3, and the number of autophagosomes with green fluorescence and those with red fluorescence only were observed by high-intensity laser confocal microscope. In *CLN3*^{*Δex7*/*8*} human neural stem cells, the number of red fluorescence single-positive autophagosomes was decreased, whereas the treatment of Compound G increased the number of red fluorescence single-positive autophagosomes in the cells (Figure 17), suggesting that the treatment of Compound G rescued the autophagic flux defect in *CLN3*^{*Δex7*/*8*} human neural stem cells.

In addition, the expression level of p62 protein was examined. As a downstream gene of the *NRF2* gene, The p62 protein expressed by *p62* is a cellular autophagy-associated protein responsible for attaching autophagic substrates to LC3 proteins which are located on the membrane of autophagosomes. Since p62 can be transported with autophagosomes to lysosomes and degraded, the intensity of intracellular autophagic flux can be characterized by comparing the changes in the amount of intracellular p62 protein after treatment with the lysosomal inhibitor Baf-A1 (p62 (Baf-A1-treated group) - p62 (Baf-A1-untreated group)). By p62 protein blotting, changes in autophagic flux within *CLN3*^{*Δex7*/*8*} human neurons were examined after treatment with compound G. The increases of p62 protein in *CLN3*^{*Δex7*/*8*} human neurons were found to be less than those of wild-type neurons after treatment with Baf-A1 (Figure 18 (a, b)); whereas treatment with Compound G followed by adding Baf-A1 resulted in higher increases of p62 protein in *CLN3*^{*Δex7*/*8*} human neurons, reaching a similar level of increase as that in wild-type neurons (Figure 18 (a, b)). These data further suggest that Compound G treatment can increase autophagic flux within neurons.

Damaged mitochondria within a cell can be eliminated through an autophagic process named mitochondrial autophagy. Functional defects in autophagy affect the elimination of damaged mitochondria, which in turn disrupts mitochondrial homeostasis in neurons. After inducing mitochondrial damage in wild-type and *CLN3*^{*Δex7*/*8*} neurons using the mitochondrial uncoupler CCCP, and then inhibiting the elimination of damaged mitochondria by Baf-A1, the increases of intracellular mitochondrial number (mtDNA/nDNA) after Baf-A1 treatment can represent the intracellular mitochondrial autophagy level. The results showed that in wild-type human neurons, the treatment of Baf-A1 significantly increased the number of intracellular mitochondria, whereas in *CLN3*^{*Δex7*/*8*} neurons, the treatment of Baf-A1 had almost no effect on the number of mitochondria (Figure 18 (c)). In contrast, when *CLN3*^{*Δex7*/*8*} neurons were treated with compound G, Baf-A1 could significantly increase the number of mitochondria (Figure 18 (c)), suggesting that compound G could rescue mitochondrial autophagy and increase the elimination efficiency of damaged mitochondria in *CLN3*^{*Δex7*/*8*} neurons.

To test the effect of compound G on mitochondrial homeostasis in *CLN3*^{*Δex7*/*8*} neurons, fluorescently labeled mitochondrial outer membrane protein OMP25-GFP was overexpressed in neurons, and the morphological changes of mitochondria were observed by laser confocal microscope. The results showed that a large number of damaged punctate mitochondria were found in *CLN3*^{*Δex7*/*8*} neurons, and Compound G could significantly reduce the occurrence of damaged punctate mitochondria (Figure 19 (a)). The amount of ATP generation in the same number of neurons was tested using Cell Titer Glo reagent. The results showed that the capacity of ATP generation in *CLN3*^{*Δex7*/*8*} neurons was significantly decreased, whereas treatment with Compound G significantly increased the capacity of ATP generation in *CLN3*^{*Δex7*/*8*} neurons (Figure 19 (b)).

Taken together, these data indicate that Compound G significantly increased the level of autophagy and mitochondrial autophagy in *CLN3*^{*Δex7*/*8*} neurons and maintained the function and homeostasis of mitochondria.

### Example 6: Compound G improved the disease phenotype of JNCL mice

The *Cln3* knockout (*Cln3 KO*) JNCL mouse model was purchased from Jackson Lab.

### Compound G improved the disease-associated phenotypes of JNCL mouse model

### A. Compound G could penetrate the blood-brain barrier

Compound G was tested firstly whether it could penetrate the mice's blood-brain barrier: it was added to the drinking water of *Cln3 KO* mice, the mice were anesthetized at different time points. After using dPBS to completely perfuse the systemic blood, the mice were euthanized and the brain tissues thereof were taken out. The drug concentrations of Compound G in the mouse brain tissues were measured. The results showed that the presence of Compound G could be detected in the mouse brain tissues obtained at different time points, and its average concentration in the mouse brain was 814.74 ± 834.19ng/g brain tissue (Figure 20). That is, Compound G could penetrate the blood-brain barrier.

### B. Compound G could reduce the abnormal storage of the proteins in the brain of JNCL mouse model

Starting from day 21 on which the mice were weaned, Compound G or the solvent was added to the drinking water of *Cln3 KO* mice, and the weight changes of the mice were recorded. It was found that when the mice were 2 or 6 months old, the body weights of *Cln3 KO* mice were not significantly different from those of the wild-type mice. At the same time, Compound G had no significant effect on the body weight of mice (Figure 21 (a)). When the mice reached six months of age, they were euthanized and the brain tissues were taken out and weighed. The data showed that at 6 months of age, the mass of the brain tissue of *Cln3 KO* mice was not significantly different from that of the wild-type mice. At the same time, Compound G had no significant effect on the mass of the mice brain tissue (Figure 21 (b)). This showed that *Cln3 KO* mice were not significantly different from the wild-type mice of the same age in terms of body weight and size of the brain tissue. Meanwhile, under the concentration used, using Compound G to conduct long-term and sustained treatment on *Cln3 KO* mice would not produce significant toxicity.

A large number of abnormally stored substances existed in the brain tissues of *Cln3 KO* mice. 2-month-old mice were perfused and euthanized according to the above method, and the brain tissues were taken out for frozen slicing and immunohistochemical staining of SCMAS. It was found that compared with the wild-type mice, the storage of a large number of SCMAS protein has appeared in the brain tissues of 2-month-old *Cln3 KO* mice (Figure 22). After *Cln3 KO* mice were weaned at 21 days of age, treatment with Compound G was initiated until the mice reached 2 months of age. It was found that treatment with Compound G could alleviate the amount of the substances abnormally stored in the brains of *Cln3 KO* mice to a certain extent (Figure 22). This demonstrated that Compound G could improve the pathological phenotypes associated with JNCL disease in *Cln3 KO* mice.

### C. Compound G could improve the learning and memory ability of JNCL mice

Starting from day 21 on which the *Cln3 KO* mice were weaned, treatment of Compound G was continuously provided. When the mice reached 5 months of age, the wild-type mice, the non-dosed *Cln3 KO* mice and the Compound G-treated *Cln3 KO* mice of the same age were taken out, followed by conducting the Morris water maze test.

First, the mice were trained in the water maze for 6 days, and the time it took for them to find the underwater platform was recorded. On the 7th day, the underwater platform was removed and the mice were tested for 60 seconds. The time that the mice spent reaching the position of the original platform for the first time, the number of shuttles at the position of the original platform and the time stayed in the quadrant of the original platform were recorded, which characterized whether they have learned the position of the underwater platform through the first 6 days of training.

The experimental results showed that wild-type mice, *Cln3 KO* mice and Compound G-treated *Cln3 KO* mice performed similarly on the first day of training, and could hardly find the underwater platform within the 60 seconds period of training (Figure 23 (a)). With the progress of learning, the time it took for the wild-type mice to find the underwater platform gradually shortened. In contrast, it took longer time for the *Cln3 KO* mice to find the underwater platform, while the performance of the Compound G-treated *Cln3 KO* mice was somewhere in between (Figure 23 (a)).

On the 7th day of training, test was conducted on the mice for the learning and memory ability. During the test, the mouse was gently put into the water from a completely new position furthest from the original platform. The results showed that compared with the wild-type mice, the time it took for the *Cln3 KO* mice to reach the position of the original platform for the first time was significantly longer (Figure 23 (b)), even longer than the time spent on the 6th day (Figure 23 (a)), maybe this was because that on the 7th day, the mice started to search for the platform from a completely new position, which was different from the familiar starting position in the training. In contrast, treatment with Compound G could effectively shorten the time that the *Cln3 KO* mice spent reaching the position of the original platform for the first time (Figure 23(b)), indicating that the mice after treated with Compound G could better learn and memorize the position of the underwater platform.

The time that the *Cln3 KO* mice stayed in the quadrant of the original platform was significantly shorter than that of the wild-type mice (Figure 23 (c)), further indicating that their memory ability for the position of the original platform was inferior to the wild-type mice. However, after treatment with Compound G, the *Cln3 KO* mice extended the time stayed in the quadrant of the original platform to a certain extent. In addition, on the 7th day of testing, the *Cln3 KO* mice had a tendency to decrease in the number of the times they shuttle at the position of the original platform, while after treatment with Compound G, the number of the times they shuttle at the position of the original platform could be closer to that of the wild-type mice (Figure 23 (d)).

In order to rule out that the *Cln3 KO* mice had defects in swimming ability in water rather than in learning and memory ability, the movement speeds of the wild-type mice and the *Cln3 KO* mice in water were counted, it was found that there was no significant difference between them. At the same time, treatment with Compound G had no significant effect on the movement speed of the mice in water (Figure 23 (e)).

The above data collectively indicated that compared with the wild-type mice, 5-month-old *Cln3 KO* mice had defects in learning and memory ability, while treatment with Compound G could improve the learning and memory ability of the *Cln3 KO* mice to a certain extent.

### D. Compound G could improve the motor ability of JNCL mice

Clinically, loss of motor ability was also a common disease phenotype in JNCL patients. Existing studies had shown that in the JNCL mouse model with the mutation of *Cln3* gene, deficits in motor ability exhibited by the patients could be observed. Therefore, the motor ability of the *Cln3 KO* mice was tested using the mine field experiment and the roller experiment, and the effect of Compound G on the motor ability of the *Cln3 KO* mice was detected.

First, the roller experiment was conducted. Treatment of Compound G was continuously provided to the mice after weaned on day 21. When the wild-type mice, the *Cln3 KO* mice and the Compound G-treated *Cln3 KO* mice reached 4-5 months of age, roller training for 3 days was conducted, and test was conducted on the 4th day for the motor ability of the mice on the roller.

Experimental data showed that on the first day of the roller experimental training, the time when the *Cln3 KO* mice first fell from the roller was earlier than that of the wild-type mice, indicating that there may be deficits in the motor ability of the *Cln3 KO* mice, while after the *Cln3 KO* mice were treated with Compound G, the fall time thereof was somewhere in between (Figure 24(a)). After 3 days of training, the falling time from the roller of the wild-type mice, the *Cln3 KO* mice and the Compound G-treated *Cln3 KO* mice all gradually extended (Figure 24(a)). On the 4th day of testing, the falling time from the roller of the *Cln3 KO* mice was significantly earlier than that of the wild-type mice, while treatment with Compound G significantly extended the falling time from the roller of the *Cln3 KO* mice, and reached a level close to that of the wild-type mice (Figure 24 (b)). This demonstrated that Compound G could alleviate the motor deficits of the *Cln3 KO* mice in the roller experiment.

Mine field experiments were conducted on 4-5 months old wild-type mice, *Cln3 KO* mice, and Compound G-treated *Cln3 KO* mice. The mouse was gently placed in the mine field, it was allowed to move freely, and the route, distance and speed of its movement within 10 minutes were recorded. The data showed that compared with the wild-type mice, the *Cln3 KO* mice had a significant decrease in the distance and the speed of the movement in the mine field, while the treatment of Compound G could improve the distance and the speed of the movement of the *Cln3 KO* mice in the mine field to a certain extent (Figure 24 (c, d)). This showed that Compound G could alleviate the motor deficits of the *Cln3 KO* mice in the mine field experiment to a certain extent.

### Example 7: Compound G inhibited KEAP1 and enhanced the functions of autolysosome

### The known targets of Compound G were barely expressed in nerve cells

Existing studies on Compound G showed that it could increase the transcription level of the transcription factor EB (TFEB) and the autophagy-related genes and the lysosomal genes by inhibiting the activities of the known target farnesoid X receptor (FXR). Compound G was added to the mouse tail tip fibroblasts (TTF), after incubation for 6 hours, mRNA was extracted, and the transcription level of the autophagy-related genes and the lysosomal genes in the cells was detected. The results showed that treatment with Compound G could significantly increase the transcription level of the autophagy-related genes and the lysosomal genes in TTF (Figure 25 (a, b)).

However, in the mouse neural stem cells, there was no significant change in the expression level of the autophagy and lysosome-related genes of the cells after treating with Compound G for 6 hours (Figure 26 (a)). Compound G did not significantly increase the transcription level of the autophagy-related genes or the lysosomal genes in the mice's neurons either (Figure 26 (b)). It was speculated from this that the known target *FXR* of Compound G was expressed at low or no levels in neural lineage cells. Transcriptome sequencing data of the wild-type and the *CLN3*^{*Δex7*/*8*} human neural stem cells confirmed that the RPKM number of *FXR* in human neural stem cells was 0.496 ± 0.573, almost no expression.

The effect of Compound G on the transcription level of the autophagy-related genes and the lysosomal genes was further detected in the induced *CLN3*^{*Δex7*/*8*} human neural stem cells and neurons. The results showed that in *CLN3*^{*Δex7*/*8*} human neural stem cells, the treatment with Compound G did not significantly increase the transcription level of the autophagy-related genes or the lysosomal genes (Figure 27 (a)). Similarly, in *CLN3*^{*Δex7*/*8*} human neurons, the treatment with Compound G did not significantly increase the expression of the autophagy-related genes or the lysosomal genes overall either (Figure 27 (b)).

To verify whether the transcriptional activation of the autophagy-related genes and the lysosomal genes by Compound G in mouse TTF was dependent on *FXR,* shRNA was used to knock down the level of *FXR* in TTF. After testing the efficiency of the knockdown of *FXR* by shRNA, shRNA-2 with the best knockdown efficiency was selected for subsequent experiments (Figure 28 (a)).

First, lentivirus packaged with control shRNA or *FXR* shRNA-2 was used to infect the mouse TTF. After treatment with Compound G, mRNA was extracted for detection of gene transcription. It was found that Compound G could significantly increase the expression of the autophagy-related genes in control shRNA-infected cells, while in TTF infected with *FXR* shRNA-2, treatment with Compound G failed to increase the expression of these genes (Figure 28 (b)). The data showed that the knockdown of FXR itself could significantly increase the expression level of *Tfeb* and autophagy-related genes (Figure 28 (b)).

The above results collectively indicated that the transcriptional activation of autophagy-related genes by Compound G was dependent on FXR.

### Compound G activated the expression of NRF2 downstream genes

The results of the aforementioned experiments showed that in human neural stem cells and neurons, Compound G could significantly increase the autophagic flux and enhance the functions of the lysosomes. It was speculated from this that in human neural stem cells and human neurons, Compound G enhanced the autophagic flux and the lysosomal functions of the cells through a completely new FXR-independent mechanism.

In order to explore the mechanism of Compound G in human neural stem cells, transcriptome sequencing experiments were conducted on wild-type human neural stem cells, *CLN3*^{*Δex7*/*8*} human neural stem cells and *CLN3*^{*Δex7*/*8*} human neural stem cells after treated with Compound G for 6 hours, and differentially expressed genes were analyzed. PCA analysis showed that in *CLN3*^{*Δex7*/*8*} human neural stem cells, treatment with Compound G for 6 hours did not produce a significant impact on the gene expression profile thereof. *CLN3*^{*Δex7*/*8*} human neural stem cells treated and untreated with Compound G were clustered together (Figure 29 (b, c)). When the genes with the fold change of the expression level > 2 and p < 0.05 after the correction were regarded as differential genes, it was found that only 3 differential genes were produced after the treatment with Compound G for 6 hours (Figure 29 (a)), namely *NQO1, LPCAT1* and *HLA-E,* their expression levels were all increased. With regard to the RNA sequencing results, the effects of Compound G on the transcription level of *TFEB,* autophagy-related genes and lysosomal genes were also analyzed. Consistent with the previous experimental results, Compound G in the *CLN3*^{*Δex7*/*8*} human neural stem cells had no significant effects on the transcription level of *TFEB* and most autophagy-related genes and lysosomal genes (Figure 30).

Analysis was conducted on the transcription level of the downstream genes of NRF2 protein in the RNA sequencing data. The results showed that after the treatment with Compound G, the expressions of multiple NRF2 downstream genes in the *CLN3*^{*Δex7*/*8*} human neural stem cells were elevated (Figure 31). Except for *NQO1,* the differential expression folds of other genes were less than 2.

Primers were further designed, verification of qRT-PCR was conducted on the above activated genes in the downstream of NRF2. After treating *CLN3*^{*Δex7*/*8*} human neural stem cells with Compound G for 6 hours, the cells were collected and mRNA was extracted for detecting the expression level of the genes. The results showed that in *CLN3*^{*Δex7*/*8*} human neural stem cells, treatment with Compound G could significantly increase the expression level of most NRF2 downstream genes (Figure 32).

The above data demonstrated that in *CLN3*^{*Δex7*/*8*} human neural stem cells, short-term treatment with Compound G could activate the expression of NRF2 downstream genes. It could be determined from this that Compound G directly activated NRF2.

### Compound G inhibited KEAP1 protein activity

Existing studies showed that the activity of NRF2 protein was mainly regulated by KEAP1 protein. Multiple electrophiles or electrophile precursor compounds could increase NRF2 activity by inhibiting KEAP1, such as isothiocyanates, α,β-unsaturated carbonyl compounds, phenols and polyphenolic compounds, etc. The basic action mode involved covalent reactions with the cysteine residues on KEAP1, mainly including cysteine residues at the positon on 151, 272 and 288 on KEAP1, resulting in changes in the binding mode of KEAP1 and NRF2, thereby activating NRF2 (Wells, 2015).

Compound G contained an α,β-unsaturated carbonyl group, and a quantitative thiol reactivity analysis was conducted to determine whether Compound G could covalently bind to the cysteine on KEAP1. After treating *CLN3*^{*Δex7*/*8*} human neural stem cells with Compound G for 2 hours, the cells were lysed, mass spectrometry was used to analyze the thiol reactivity of the cysteine residues on KEAP1 in the lysate. The results showed that treatment with Compound G could reduce the thiol reactivity of cysteine residue 288 of the KEAP1 protein (Figure 33), indicating that Compound G may inhibit the functions of KEAP1 by covalently binding to the cysteine residue 288 of the KEAP1 protein, thereby activating the expression of NRF2 and its downstream genes.

To verify whether inhibition of KEAP1 could alleviate the JNCL disease phenotype, shRNA targeting *KEAP1* was used to knock down the KEAP1 protein in the *CLN3*^{*Δex7*/*8*} human neuron model. Five shRNA lentiviral plasmids targeting *KEAP1* were selected from the human shRNA library, followed by using HEK293T cells to package them into lentivirus, and infecting the human neural stem cells. To detect the knockdown efficiency of shRNA, mRNA was extracted, and qRT-PCR was used to detect the relative abundance of the *KEAP1* mRNA in cells. The results showed that *KEAP1* shRNA-3 had the highest knockdown efficiency (Figure 34), then this shRNA was used to conduct subsequent experiments, detecting the impact of the knockdown of *KEAP1* on the *CLN3*^{*Δex7*/*8*} human neurons.

First, it was tested whether knockdown of *KEAP1* could achieve similar effects. Lysotracker staining was used to indicate the acidity of the lysosomes in cells, and flow cytometry was used to analyze the average fluorescence intensity of the staining. The Lysotracker average fluorescence intensity of the *KEAP1* -knockdown *CLN3*^{*Δex7*/*8*} human neurons was significantly enhanced, while adding Compound G to the *KEAP1* -knockdown *CLN3*^{*Δex7*/*8*} human neurons could not further increase the Lysotracker average fluorescence intensity (Figure 35). This data demonstrated that knockdown of *KEAP1* could increase the lysosomal acidity in *CLN3*^{*Δex7*/*8*} human neurons, while the ability of Compound G to increase the lysosomal acidity was dependent on *KEAP1.*

Next, the impact of the knockdown of *KEAP1* on the activity of the protease in the lysosome of the *CLN3*^{*Δex7*/*8*} human neural stem cells was tested. A substrate for cathepsin B was added to the cells, this substrate could emit red fluorescence after cleaved by cathepsin B. Cell imaging was conducted using Opera high-content laser confocal microscopy. The results showed that compared with the wild-type neural stem cells, the red fluorescence intensity emitted after cathepsin B cleaving the substrate was significantly reduced in *CLN3*^{*Δex7*/*8*} cells, while the knockdown of *KEAP1* could significantly increase the intracellular red fluorescence intensity (Figure 36 (a, b)). The above data demonstrated that the knockdown of *KEAP1* could increase the protease activity in the lysosomes of the *CLN3*^{*Δex7*/*8*} cells. The storage of SCMAS protein in the KEAP1-knockdown *CLN3*^{*Δex7*/*8*} human neurons was also significantly reduced (Figure 37).

To further detect the impact of the knockdown of *KEAP1* on the intracellular autophagic flux, tandem LC3 protein was overexpressed in human neural stem cells to mark the intracellular autophagosomes, and imaging was conducted using the high-content laser confocal microscopy. The results showed that, similar to Compound G, the *KEAP1* knockdown could significantly increase the number of the red-fluorescence single-positive autophagosomes in the *CLN3*^{*Δex7*/*8*} human neural stem cells. This result suggested that the *KEAP1* knockdown could effectively alleviate the defect of the autophagic flux in the *CLN3*^{*Δex7*/*8*} human neural stem cells (Figure 38 (a, b)).

Next, the impact of the knockdown of *KEAP1* on the homeostasis of the *CLN3*^{*Δex7*/*8*} human neurons was detected. The OMP25-GFP lentiviral plasmid was used to infect the *KEAP1* -knockdown *CLN3*^{*Δex7*/*8*} human neurons to mark the morphology of mitochondria in the cells. Then cell imaging was conducted using the laser confocal microscopy. There were a large number of damaged punctate mitochondria in *CLN3*^{*Δex7*/*8*} human neurons, while treatment with Compound G or knockdown of *KEAP1* could both significantly reduce the appearance of the damaged mitochondria in *CLN3*^{*Δex7*/*8*} human neurons (Figure 39).

The impact of the knockdown of *KEAP1* on the ATP production capacity in *CLN3*^{*Δex7*/*8*} human neurons was also detected. The results showed that the knockdown of *KEAP1* could significantly increase the ability of the *CLN3*^{*Δex7*/*8*} human neurons to generate ATP (Figure 40). The above results demonstrated that, the knockdown of *KEAP1* could help maintain the homeostasis and the functions of the mitochondria in *CLN3*^{*Δex7*/*8*} human neurons, which is consistent with the treatment effect of Compound G.

In summary, in the *CLN3*^{*Δex7*/*8*} human cell model, the knockdown of *KEAP1* could mimic the effects of Compound G on lysosomal functions, autophagic flux intensities, and mitochondrial homeostasis. Compound G exerted the above functions in a KEAP1-dependent manner.

### Compound G enhanced the lysosomal acidity through the p62-KEAP1 pathway

The present disclosure detected the impact of the Compound G treatment or the KEAP1 knockdown on the intracellular p62 protein level. Compound G was added to human neurons, after incubation for 24 hours, the cell lysate was extracted, qRT-PCR was used to detect the transcription level of the *p62* gene. The results showed that treatment with Compound G or knockdown of KEAP1 could significantly increase the transcription level of *p62* in human neurons (Figure 41 (a)). In human neurons, treatment with Compound G or knockdown of KEAP1 could also increase the p62 protein level (Figure 41 (b)). It had been demonstrated above that Compound G could still significantly increase the intracellular protein level of p62 under the condition of adding Baf-A1, which indicated that Compound G regulated the amount of p62 protein not by inhibiting the functions of lysosomes (Figure 22 (a, b)).

The impact of increased p62 protein levels on the cellular lysosomal functions was detected: GFP-p62 proteins and GFP proteins of the control group were overexpressed in NRK cells, and Lysotracker dye was used to detect the acidity of lysosomes in GFP-positive cells. The results of the flow cytometry analysis showed that, similar to Compound G, the overexpression of p62 proteins could significantly increase the fluorescence intensity of the intracellular Lysotracker dye (Figure 42 (a)), indicating that the overexpression of p62 proteins could increase the intracellular lysosomal acidity.

Treatment with dimethyl fumarate (DMF), another known KEAP1 inhibitor, could also significantly increase the intracellular lysosomal acidity (Figure 42 (a)). At the same time, a similar phenomenon was also observed after overexpressing GFP-p62 proteins in human neural stem cells, overexpression of p62 proteins could significantly increase the lysosomal acidity in neural stem cells (Figure 42 (b)), and alleviate the abnormal accumulation of toxic proteins in *CLN3*^{*Δex7*/*8*} human neurons and the neuronal death (Figure 43).

The above results collectively indicated that the inhibition of KEAP1 increased the lysosomal acidity by increasing the intracellular NRF2 downstream proteins such as the protein level of p62.

### Example 8: Compound G reduced the accumulation of toxic proteins in human neuronal model of Alzheimer's dementia

The present disclosure detected the therapeutic effect of the treatment of Compound G on Alzheimer's dementia. We directed the differentiation of human embryonic stem cells with mutations in APP gene into human neurons in vitro and constructed an in vitro human neuron model of Alzheimer's dementia. This model exhibited the deposition of the extracellular Aβ plaques and the increased levels of toxic Aβ42 secreted by the cells into the culture medium. APP-gene mutant human neurons after treatment with Compound G showed a significant reduction in the deposition of Aβ plaques and a significant reduction in the level of toxic Aβ42 secreted by the cells into the culture medium (Figure 44).

### Conclusion

The present disclosure reveals that small molecule compounds including Compound G enhance the autophagic flux and the lysosomal functions in human neurons by inhibiting KEAP1, thereby benefiting the treatment of JNCL. It is demonstrated at the same time that knockdown of the KEAP1 gene can also achieve the same effects. In addition to JNCL, lysosomal functional deficiency and autophagy functional reduction are also involved in the pathogenesis of a variety of NCLs and other late-onset neurodegenerative diseases. It is reasonable to infer that inhibiting the activity of KEAP1 also has certain therapeutic effects on other neurodegenerative diseases related to autophagy and lysosomal functional deficiencies. Means of inhibiting KEAP1 include using small molecule compounds identified in the present disclosure including Compound G or knocking down the KEAP1 gene.

Although specific embodiments of the present disclosure have been combined to describe the present disclosure, it is evident that many alternatives, modifications and variations are obvious for those skilled in the art. Correspondingly, the present disclosure embraces all such alternatives, modifications and variations that fall within the spirit and scope of the appended claims. All publications, patents and patent applications mentioned in the present specification are all incorporated into this specification in their entirety by reference in the present specification.

## Claims

1. A method for treating a disease associated with autophagy functional deficiency and/or lysosomal functional deficiency, comprising a process of inhibiting the activity of Kelch-like ECH-associated protein 1 (KEAP1), and/or enhancing the activity of nuclear erythroid 2-related factor 2 (NRF2), and/or the downstream proteins thereof.

2. The method according to claim 1, wherein the disease associated with autophagy functional deficiency and/or lysosomal functional deficiency is a neurodegenerative disease.

3. The method according to claim 2, wherein the neurodegenerative disease is selected from the group consisting of amyotrophic lateral sclerosis (ALS), Alzheimer's dementia, Alexander disease, Alper's disease, ataxia-telangiectasia, bovine spongiform encephalopathy (BSE), Canavan disease, Cockayne syndrome, corticobasal degeneration, Creutzfeldt-Jakob disease, Huntington's disease, HIV-related dementia, Kennedy's disease, Krabbedisease, dementia with Lewy bodies, Machado-Joseph disease (spinocerebellar ataxias 3), multiple sclerosis, multiple system atrophy, neurospirillosis, Parkinson's disease, Péme's disease, Pick's disease, primary lateral sclerosis, Prion's disease, Refsum's disease, Sandhoff disease, Hield's disease, schizophrenia, Spielmeyer-Vogt-Sjogren-Batten disease, spinocerebellar ataxias, spinal muscular atrophy, or neuronal ceroid lipofuscinosis (NCL), preferably the neuronal ceroid lipofuscinosis isjuvenile neuronal ceroid lipofuscinosis (JNCL).

4. The method according to claim 3, wherein the disease is caused by a mutation in CLN3 gene, such as caused by a deletion mutation in exons 7 and 8 of CLN3 gene.

5. The method according to any one of claims 1 to 4, wherein the expression of KEAP1 gene is silenced or knocked down with siRNA, sgRNA or vector constructed with shRNA, thereby enhancing the activity of NRF2 and/or the downstream proteins thereof.

6. The method according to any one of claims 1 to 4, wherein the expression of NRF2 and/or the downstream gene thereof is enhanced with a KEAP1 inhibitor and/or NRF2 activator, thereby enhancing the activity of NRF2 and/or the downstream proteins thereof.

7. The method according to claim 6, wherein the KEAP1 inhibitor and/or NRF2 activator is selected from reagents covalently reacting with cysteine residues on KEAP1, including cysteine at the positon 151, 272 and/or 288 on KEAP1.

8. The method according to claim 6, wherein the KEAP1 inhibitor and/or NRF2 activator is selected from the group consisting of Carvedilol (CAS: 72956-09-3), ketoconazole (CAS: 65277-42-1), GANT61, CAS (500579-04-4), protriptyline hydrochloride (CAS: 1225-55-4), LP 44 (CAS: 824958-12-5), Doxepin HCI (CAS: 1229-29-4), dimethyl fumarate (DMF), acetyl-11-keto-β-boswellic acid (AKBA), isothiocyanates, bardoxolone (CDDO), bardoxolonemethyl (CDDO-Me) and the derivatives or analogues thereof, or selected from one or more of α, β-unsaturated carbonyl compounds, phenols and polyphenolic compounds.

9. The method according to claim 6, wherein the KEAP1 inhibitor is Compound G, namely (Z)-guggulsterone, having the formula:

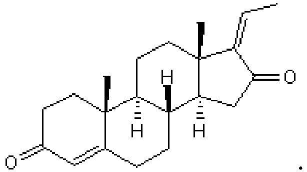

10. A pharmaceutical composition for treating a disease associated with autophagy functional deficiency and/or lysosomal functional deficiency, comprising a therapeutically effective amount of one or more of KEAP1 inhibitor and/or NRF2 activator, and a pharmaceutically acceptable carrier and/or excipient.

11. The pharmaceutical composition according to claim 10, wherein the KEAP1 inhibitor and/or NRF2 activator is selected from reagents covalently reacting with cysteine residues on KEAP1, including cysteine at the positon 151, 272 and/or 288 on KEAP1.

12. The pharmaceutical composition according to claim 10, wherein the KEAP1 inhibitor and/or NRF2 activator is selected from the group consisting of Carvedilol (CAS: 72956-09-3), ketoconazole (CAS: 65277-42-1), GANT61, CAS (500579-04-4), protriptyline hydrochloride (CAS: 1225-55-4), LP 44 (CAS: 824958-12-5), Doxepin HCI (CAS: 1229-29-4), dimethyl fumarate (DMF), acetyl-11-keto-β-boswellic acid (AKBA), isothiocyanates, bardoxolone (CDDO), bardoxolonemethyl (CDDO-Me) and the derivatives or analogues thereof, or selected from one or more of α, β-unsaturated carbonyl compounds, phenols and polyphenolic compounds.

13. The pharmaceutical composition according to claim 10, wherein the KEAP1 inhibitor is Compound G, namely (Z)-guggulsterone, having the formula:

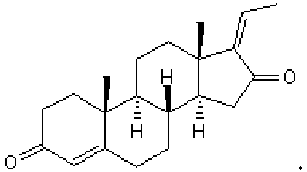

14. A kit for treating a disease associated with autophagy functional deficiency and/or lysosomal functional deficiency, comprising a reagent for inhibiting KEAP1 gene expression.

15. The kit according to claim 14, wherein the kit comprises siRNA, sgRNA or a vector constructed with shRNA.

16. A method for alleviating or eliminating the differentiation defect of neural stem cells into neurons in a subject, comprising administering a medicament inhibiting KEAP1 activity and/or enhancing the activity of NRF2 and/or downstream proteins thereof to a subject in need, wherein the subject is suffering from a disease associated with autophagy functional deficiency and/or lysosomal functional deficiency.

17. The method of claim 16, wherein the disease is caused by a mutation in CLN3 gene, such as caused by a deletion mutation in exons 7 and 8 of CLN3 gene.

18. The method according to claim 16 or 17, wherein the medicament is selected from one or more of the following medicaments: Compound G, namely (Z)-guggulsterone, Carvedilol (CAS: 72956-09-3), Ketoconazole (CAS: 65277-42-1), GANT61, CAS (500579-04-4), Protriptyline hydrochloride (CAS: 1225-55-4), LP 44 (CAS: 824958-12-5), Doxepin HCl (CAS: 1229-29-4), dimethyl fumarate (DMF), acetyl-11-keto-β-boswellic acid (AKBA), isothiocyanates, bardoxolone (CDDO), bardoxolonemethyl (CDDO-Me) and the derivatives or analogues thereof, or selected from other α, β-unsaturated carbonyl compounds, phenols and polyphenolic compounds, preferably Compound G, namely (Z)-guggulsterone.

19. A method for screening substances activating autophagic flux and/or enhancing lysosomal function, wherein a Tandem LC3 reporter system comprising dual fluorescent labels is used;in the system, when autophagosomes with the dual fluorescently labeled LC3 is located in the cytoplasm, the autophagosomes present dual fluorescence; when autophagosomes fuse with lysosomes to form an autolysosome, the autophagosomes only present single fluorescence;
if a screened substance increases the number of the dual fluorescence labeled autophagosomes and the number of single fluorescence labeled autolysosomes in the reporter system, then the substance is a target substance activating autophagic flux and/or enhancing lysosomal function.

20. An *in vitro* model based on neural stem cells or neurons comprising a deletion mutation of exons 7 and 8 of the CLN3 gene, namely *CLN3*^{*Δex7*/}*⁸.*

21. Use of Compound Gnamely (Z)-guggulsterone in the manufacture of a medicament for treating diseases or conditions caused by CLN3 gene mutations, preferably, the CLN3 gene mutations refer to deletion mutations of exons 7 and 8.
